(19) **Europäisches Patentamt / European Patent Office / Office européen des brevets**

(11) **EP 2 314 306 A1**

(12) **EUROPEAN PATENT APPLICATION**

(43) Date of publication:
**27.04.2011 Bulletin 2011/17**

(51) Int Cl.:
*A61K 38/17* $^{(2006.01)}$      *C07K 14/705* $^{(2006.01)}$

(21) Application number: **10180543.0**

(22) Date of filing: **29.04.2002**

(84) Designated Contracting States:
**AT BE CH CY DE DK ES FI FR GB GR IE IT LI LU MC NL PT SE TR**

(30) Priority: **27.04.2001 US 287088 P**

(62) Document number(s) of the earlier application(s) in accordance with Art. 76 EPC:
**02766877.1 / 1 409 510**

(71) Applicant: **Johns Hopkins University Baltimore, MD 21202 (US)**

(72) Inventors:
• **Marban, Eduardo**
  **Lutherville, MD 21152 (US)**
• **Li, Ronald, A.**
  **Baltimore, MD 21237 (US)**

(74) Representative: **Hiebl, Inge Elisabeth**
  **Kraus & Weisert**
  **Patent- und Rechtsanwälte**
  **Thomas-Wimmer-Ring 15**
  **80539 München (DE)**

Remarks:
This application was filed on 28-09-2010 as a divisional application to the application mentioned under INID code 62.

(54) **Biological pacemaker**

(57)    Disclosed are methods and systems for modulating electrical behavior of cardiac cells. Preferred methods include administering a polynucleotide or cell-based composition that can modulate cardiac contraction to desired levels, i.e., the administered composition functions as a biological pacemaker.

EP 2 314 306 A1

## Description

The present application claims priority to United States Provisional Application No. 60/287,088 filed on April 27,2001, which is incorporated by reference herein in its entirety.

STATEMENT OF FEDERALLY SPONSORED RESEARCH

Funding for the present invention was provided in part by the Government of the United States by virtue of Grant No. NIH P50 HL52307 by the National Institutes of Health. Thus, the Government of the United States has certain rights in and to the invention claimed herein.

BACKGROUND

1. Field of the Invention.

The invention generally features methods to provide and/or modulate a cardiac pacemaker function. In preferred aspects, the invention provides genetically-engineered pacemakers that can be employed as an alternative or supplement to implantable electronic pacemakers to induce or modulate ventricular or atrial firing rate.

2. Background.

Spontaneous cellular electrical rhythms govern numerous biological processes from the autonomous beating of the heart, to respiratory rhythms and sleep cycles.
In particular, the mammalian heart is understood to maintain an intrinsic rhythm by creating electric stimuli. Generally, the stimuli form a depolarization wave that originates in so-called pacemakers and then propagates within specialized cardiac conducting tissues and the myocardium. The usually well-ordered wave movement facilitates coordinated contractions of the myocardium. These contractions are the engine that moves blood throughout the body. See generally The Heart and Cardiovascular System. Scientific Foundations. (1986) (Fozzard, H.A. et al. eds) Raven Press, NY.
Under most circumstances, cardiac stimuli are controlled by recognized physiological mechanisms. However there has been long-standing recognition that abnormalities of excitable cardiac tissue can lead to abnormalities of the heart rhythm.
Such heart rhythm abnormalities are associated with various diseases and disorders that are significant and pervasive throughout the United States. See Bosch, R. et al. (1999) in Cardiovas Res. 44: 121 and references cited therein. For instance, bradyarrhythmias result in greater than 255,000 electronic pacemaker implants per year in the United States.
Traditional treatment methods have included implantable (electronic) pacemakers that deliver a fixed or variable frequency of pacing pulses to the patient's heart. However, such implanted devices have significant inherent risks such as infection, hemorrhage, lung collapse as well as significant expense.
It thus would be desirable to have new methods to provide desired rate of cardiac contraction (firing rate).

SUMMARY OF THE INVENTION

We now provide gene transfer and cell administration methods that can create a pacemaker function, and/or modulate the activity of an endogenous or induced cardiac pacemaker function.
Methods of the invention may be employed to create and/or modulate the activity of an endogenous pacemaker (such as the sinotrial node of a mammalian heart) and/or an induced pacemaker (e.g. biological pacemaker generated from stem cells or converted electrically-quiescent cells).
More particularly, in a preferred aspect of the invention, quiescent heart muscle cells are converted into pacemaker cells by *in vivo* viral gene transfer or modified cell transfer (e.g., differentiated stem cells).
In a further aspect, a composition is administered to a subject to alter the frequency of (i.e. to tune) an existing endogenous or induced cardiac pacemaker function. Polynucleotides or modified cells are preferred agents for administration.
Preferred methods of the invention include dominant-negative suppression of Kir2-encoded potassium channels in the ventricle to produce spontaneous, rhythmic electrical activity. The rate of the induced pacemakers can increase with β-adrenergic stimulation. Thus, by methods of the invention, latent pacemaker activity of ventricular myocytes can be unleashed by inhibition of Kir2 channels.
Preferred methods include administering a polynucleotide compound that can suppress activity of a Kir2 gene *in vivo.* A variety of particular approaches may be employed. For instance, an antisense compound or other polynucleotide inhibitor can be administered to the mammal that can suppress expression of a Kir2 gene. A gene knockout approach also may be employed, e.g. knockout of a Kir2 gene present in a stem cell. Any of a number of the Kir2 gene family members may be suppressed, including e.g. the Kir2.1, Kir2.2, Kir2.3 and/or Kir2.4 genes.

As mentioned, modified cells also may be administered to induce or modulate pacemaker to cells or a subject. For instance, the administered modified cells suitably may be cardiomyocytes that can provide pacemaker function and have been differentiated from stems cells such as embryonic or bone marrow stem cells. The latter may be stem cell-derived pacemaker cells (by spontaneous and/or driven differentiation) and/or stem cell-derived myocytes (e.g. ventricular cells) that have been converted to provide pacemaker function.

The modified cells may have been harvested from the recipient, i.e. the subject to which the cells are administered. For example, bone marrow stem cells may be harvested from a subject and then differentiated to cardiomyocytes with pacemaker function. Cardiac cells, such as sino-atrial node cells may be harvested from a subject such as through removal via catheter or other protocol, modified e.g. by insertion of a desired polynucleotide delivery system as disclosed herein and the transformed cells then administered to the subject. As referred to herein, the administered cells preferably are modified in some respect prior to administration, such as stem-cell-derived cardiomyocytes or cardiomyocytes that are transformed with an expression system such as those disclosed herein.

In an alternative or supplemental strategy, hyperpolarization-activated, cyclic-nucleotide gated (HCN) gene expression may be promoted by administering an appropriate polynucleotide compound to a mammal. We have particularly found that HCN activity can be modulated in both positive and negative directions such that their activation thresholds can be shifted to a desired level. Hence, cardiac pacing, and subsequent heart rate, can be effectively modulated (both increase and decrease) by such control of nucleotide gated (HCN) gene expression. For instance, pacing can be decelerated by inhibiting the endogenous HCN channel activity and/or by shifting the activation threshold above the endogenous level (alternatively, acceleration can be achieved by increasing the numbers of operating channels via overexpression and/or by shifting the activation threshold below the endogenous level). Similarly, the response of the native pacemakers to the secondary messenger cAMP also can be modulated, e.g. by using engineered channels of given sensitivities.

Particularly preferred is use of an HCN construct that is coupled with a Kir sequence in a co-expression vector. That vector can be administered to both induce pacemaker activity and to modulate or otherwise control pacemaker rate. Especially preferred is to couple an HCN construct with a Kir2.1AAA sequence in a co-expression vector.

The administered polynucleotide suitably induces or modulates (increase or decrease) at least one heart electrical property. Preferred use of the invention modulates heart electrical conduction and preferably can reconfigure all or part of the cardiac action potential (AP). That use helps achieve a desired therapeutic outcome. Significant disruption of normal electrical function is usually reduced and often avoided by the present methods.

Preferably, administration of a polynucleotide or modified cells to myocardial cells in accordance with the invention will provide a discernable difference (increase or decrease) in the rate of electrical signal output (myocardial cell or ventricular firing rate) of the treated myocardial cells. More particularly, preferably the administration provides at least about a 2, 3, 4 or 5 percent increase or decrease, more preferably at least about a 10, 15, 20, 25, 30, 40, 50 or 100 percent (increase or decrease) in the firing rate of the treated myocardial cells. Firing rate of treated cells may be determined by standard procedures, particularly by a standard electrophysiological assay as such assay is defined below.

Examples of preferred administration routes, polynucleotides, and assays are provided in the discussion that follows. In general, polynucleotide expression conducive to using the invention is apparent as a shift in a recording (relative to baseline) obtained from at least one of the standard electrophysiological assays. Preferably, administration of polynucleotide in accordance with the invention provides an increase or decrease of an electrical property by at least about 10% relative to a baseline function. More preferably, the increase or decrease is at least about 20%, more preferably at least about 30% to about 50% or more. That baseline function can be readily ascertained e.g. by performing the electrophysiological assay on a particular mammal prior to conducting the invention methods. Alternatively, related baseline function can be determined by performing a parallel experiment in which a control polynucleotide is administered instead of the polynucleotide of interest. It will be apparent that once a reliable baseline function has been established (or is available from public sources), determination of the baseline function by the practitioner may not always be necessary. Examples of relevant electrical properties are known and include, but are not limited to, at least one of heart rate, refractoriness, speed of conduction, focal automaticity, and spatial excitation pattern. Heart or contraction rate (firing rate) or pulse rate is preferably evaluated.

By modulating cardiac contraction rate, the invention can be employed to treat or prevent (prophylactic treatment) a wide range of cardiac related diseases and disorders. For example, methods of the invention will be useful for treatment of subjects suffering from or susceptible to cardiac related syncope, particularly Stokes-Adam syncope. Methods of the invention also will be useful to treat subjects suffering from or susceptible to various abnormalities of sinus node function, including persistent sinus bradycardia, sino-atrial (S-A) block manifested as S-A Wenckebach, complete S-A block or sinus arrest (sinus impulse fails to activate the atria), and high-grade atriventricular block. Methods of the invention also will be useful to treat subjects suffering from or susceptible to bradycardia-tachycardia syndrome, and bradycardia of other causes.

Therapeutic methods of the invention generally comprise administration of an effective amount of firing rate modulating composition to a mammal in accordance with the invention. The administration is preferably localized within targeted areas of cardiac tissue of the mammal to avoid e.g. toxicity. Such administration is suitably accomplished by injection, catheter delivery and other means as disclosed e.g. herein. Preferably, the mammal is first identified and selected for the treatment and then the therapeutic composition is administered. For instance, a mammal can be identified that is suffering from or susceptible to a disease or disorder as disclosed herein such as a cardiac-related syncope, particularly Stokes-Adam syncope; an abnormality of sinus node function such as persistent sinus bradycardia, sino-atrial (S-A) block manifested as S-A Wenckebach, complete S-A block or sinus arrest, and high-grade atriventricular block; or bradycardia-tachycardia syndrome or other bradycardia related condition.

In another aspect, the invention provides a kit for performing one or a combination of the invention methods disclosed herein. Preferably, the kit includes at least one suitable myocardium nucleic acid delivery system and preferably at least one desired polynucleotide and/or modified cell composition. Preferably, that polynucleotide is operably linked to the system i.e., it is in functional and/or physical association therewith sufficient to provide for good administration of the polynucleotide into the heart. Additionally preferred kits include means for administering the polynucleotide or modified cells to a mammal such as a syringe, catheter and the like.

The invention also relates to the following items:

1. A method for modulating cardiac contraction function or cardiac electrical activity, comprising administering a polynucleotide or modified cells to quiescent myocardial cells, whereby after administration the myocardial cells generate spontaneous repetitive electrical signals.

2. The method of item 1 wherein expression of the polynucleotide after administration provides at least about a ten percent change in the frequency of the electrical signal output of the cells.

3. The method of item I or 2 wherein the polynucleotide is a dominant-negative construct.

4. The method of any one of items 1 through 3 wherein the polynucleotide can suppress Kir2-encoded ion channels of the cells.

5. The method of any one of items 1 through 4 wherein the transduced myocardial cells produce spontaneous, rhythmic electrical activity.

6. The method of any one of items 1 through 6 wherein expression of the polynucleotide after administration is driven by an inducible promoter.

7. The method of any one of items I through 6 wherein the polynucleotide comprises one or more nucleic acid sequences that code for molecules which suppress inward rectifier potassium currents.

8. The method of any one of items 1 through 7 wherein the polynucleotide comprises a sequence that corresponds to a sequence of a member of the Kir2 family of genes.

9. The method of item 8 wherein the polynucleotide encodes for three alanine molecules at amino acid positions 144 to 146 as compared to a wild type Kir2 molecule.

10. The method of item 9 wherein the dominant-negative Kir2 alanine encoding molecule is co-expressed in cells expressing wild type Kir2 molecules.

11. The method of item 10 wherein the co-expression suppresses current flux as compared to cells expressing wild type Kir2 molecules.

12. The method of item 11 wherein suppression of current flux modulates cardiac contraction and/or electrical activity of a mammal.

13. The method of any one of items 1 through 12 wherein the polynucleotide comprises an inducible promoter that regulates transcription of a Kir2 nucleic acid sequence.

14. The method of item 13 wherein the inducible promoter is regulated by an externally controllable stimulus.

15. The method of item 13 wherein the inducible promoter is regulated by a hormone or cytokine.

16. The method of any one of items 1 through 15 wherein the quiescent myocardial cells are identified and selected and thereafter the polynucleotide is administered.

17. A method for modulating cardiac contraction function, comprising administering a polynucleotide or modified cells to myocardial cells that are generating electrical signals at an inappropriate frequency,
whereby after administration the myocardial cells generate electrical signals at a desired increased or decreased frequency, which is changed from the electrical signal frequency of the cells prior to the administration.

18. The method of item 17 wherein expression of the polynucleotide after administration provides at least about a ten percent change in the frequency of the electrical signal output of the cells.

19. The method of item 17 or 18 wherein the polynucleotide is a dominant-negative construct.

20. The method of any one of items 17 through 19 wherein the polynucleotide can suppress Kir2-encoded ion channels of the cells.

21. The method of any one of items 17 through 20 wherein expression of the polynucleotide after administration is driven by an inducible promoter.

22. The method of any one of items 17 through 21 wherein the polynucleotide comprises one or more nucleic acid sequences that code for molecules which suppress inward rectifier potassium currents.

23. The method of any one of items 17 through 22 wherein the polynucleotide comprises a sequence that corresponds to a sequence of a member of the Kir2 family of genes.

24. The method of item 23 wherein the polynucleotide encodes for three alanine molecules at amino acid positions 144 to 146 as compared to a wild type Kir2 molecule.

25. The method of item 23 wherein the dominant-negative Kir2 alanine encoding molecule is co-expressed in cells expressing wild type Kir2 molecules.

26. The method of item 25 wherein the co-expression suppresses current flux as compared to cells expressing wild type Kir2 molecules.

27. The method of item 26 wherein suppression of current flux modulates cardiac contraction activity of a mammal.

28. The method of any one of items 17 through 27 wherein the polynucleotide comprises an inducible promoter that regulates transcription of a Kir2 nucleic acid sequence.

29. The method of item 28 wherein the inducible promoter is regulated by an externally controllable stimulus.

30. The method of item 17 wherein the inducible promoter is regulated by a hormone or cytokine.

31. The method of any one of items 1 through 30 wherein the modified cells are stem cells.

32. The method of any one of items I through 30 wherein the cells are somatic cells.

33. The method of any one of items 1 through 30 wherein the myocardial cells are identified and selected based on electrical signal activity or frequency and thereafter the polynucleotide is administered.

34. A method of treating a mammal suffering from or susceptible to undesired cardiac contraction or cardiac electrical activity, comprising
administering to the mammal an effective amount of a composition that comprises a polynucleotide or modified cells, whereby cardiac contraction activity or cardiac electrical activity of the mammal is modulated by the administration.

35. The method of item 34 wherein the composition provides at least about a ten percent change in the frequency of the electrical signal output of myocardial cells of the mammal.

36. The method of item 34 or 35 wherein the composition can modulate an HCN and/or Kir2 channel of the mammal.

37. The method of any one of items 34 through 36 wherein the administered composition comprises a polynucleotide.

38. The method of item 37 wherein the administered composition comprises a dominant-negative construct.

39. The method of item 37 or 38 wherein the polynucleotide can suppress Kir2-encoded ion channels of the mammal.

40. The method of any one of items 37 through 39 wherein expression of the polynucleotide after administration is driven by an inducible promoter.

41. The method of any one of items 37 through 40 wherein the polynucleotide comprises one or more nucleic acid sequences that code for molecules which suppress inward rectifier potassium currents.

42. The method of any one of items 37 through 41 wherein the polynucleotide comprises a sequence that corresponds to a sequence of a member of the Kir2 family of genes.

43. The method of item 34 through 36 wherein the composition comprises modified cells.

44. The method of any one of items 34 through 43 wherein the mammal has an implanted pacemaker and administration of the composition modulates cardiac contraction rate in conjunction with the implanted pacemaker.

45. The method of any one of items 34 through 44 wherein the mammal suffering from undesired cardiac contraction is identified and selected and the composition then administered.

46. A method of treating a mammal suffering from or susceptible to cardiac related syncope, abnormal sinus node function, atriventricular block, or bradycardia-tachycardia syndrome, comprising
administering to the mammal an effective amount of a composition that a polynucleotide or modified cells,
whereby cardiac contraction activity of the mammal is modulated by the administration.

47. The method of item 46 wherein administration of the composition provides at least about a ten percent change in the frequency of the electrical signal output of myocardial cells of the mammal.

48. The method of item 46 or 47 wherein the composition can modulate an HCN and/or Kir2 channel of the mammal.

49. The method of any one of items 46 through 48 wherein the administered composition comprises a polynucleotide.

50. The method of item 49 wherein the administered composition comprises a dominant-negative construct.

51. The method of item 49 or 50 wherein the polynucleotide can suppress Kir2-encoded ion channels of the mammal.

52. The method of any one of items 49 through 51 wherein expression of the polynucleotide after administration is driven by an inducible promoter.

53. The method of any one of items 49 through 52 wherein the polynucleotide comprises one or more nucleic acid sequences that code for molecules which suppress inward rectifier potassium currents.

54. The method of any one of items 49 through 53 wherein the polynucleotide comprises a sequence that corresponds to a sequence of a member of the Kir2 family of genes.

55. The method of any one of items 46 through 54 wherein the mammal has an implanted pacemaker and administration of the composition modulates cardiac contraction rate in conjunction with the implanted pacemaker.

56. The method of item 46 through 48 wherein the modified cells are stem cells.

57. The method of any one of items 46 through 56 wherein the mammal suffering from the disorder is identified and selected and the composition then administered.

58. The method of any one of items 46 through 57 wherein a polynucleotide is administered to the mammal that encodes a K channel subunit, Na channel subunit, Ca channel subunit, an inhibitory G protein subunit, a connexin; or a functional fragment thereof.

59. The method of item 58 wherein the Ca channel subunit is a beta 1, or alpha2-delta subunit from an L-type Ca channel.

60. The method of item 58 wherein the Na channel subunit is betal or beta2.

61. The method of item 58 wherein the inhibitory G protein subunit is $G\alpha_{i2}$ or a functional fragment thereof.

62. The method of any one of items 1 through 61 wherein the administration step comprises injecting the composition into myocardium.

63. The method of any one of items 1 through 61 wherein the administration step comprises perfusing the composition into cardiac vasculature.

64. The method of any one of items 1 through 63 wherein the mammal is a human.

65. A method of treating a mammal suffering from or susceptible to undesired cardiac contraction or cardiac electrical activity, comprising
administering to the mammal an effective amount of a composition that comprises a polynucleotide or modified cells, wherein the mammal has an implanted electronic pacemaker, and the administration modifies the cardiac contraction activity or cardiac electrical activity provided by the electronic pacemaker.

66. The method of item 65 wherein the composition is administered to cardiac tissue at a site distinct from the implanted pacemaker.

67. The method of item 65 or 66 wherein a polynucleotide is administered that is a dominant-negative construct.

68. The method of any one of items 65 through 67 wherein the polynucleotide can suppress Kir2-encoded ion channels of the cells.

Other aspects of the invention are discussed infra.

BRIEF DESCRIPTION OF THE DRAWINGS

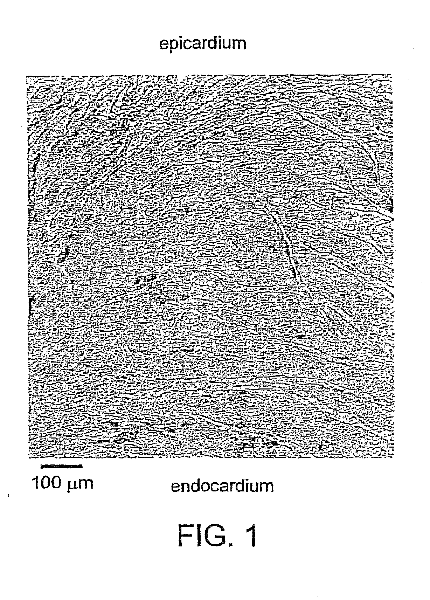

Figure 1. Assessment of gene transfer efficacy. X- gal staining of microscopic sections of left ventricle (LV) 48 hours after injection of AdCMV- gal into the LV cavity was used to assess transduction efficacy. Transduced cells (stained blue) were observed throughout the LV wall. This gene delivery method achieved transduction of 20% of ventricular myocytes without obvious cell damage.

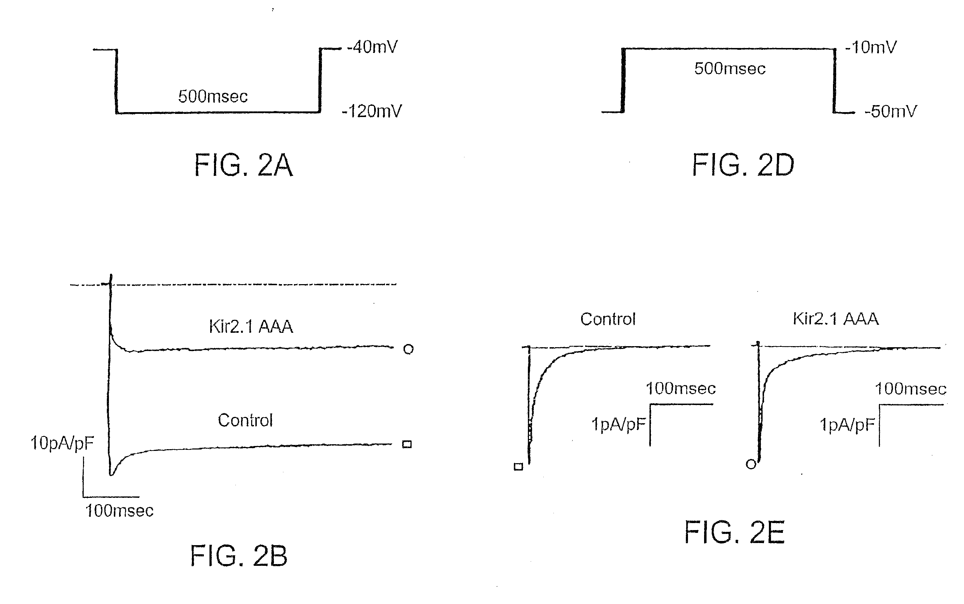

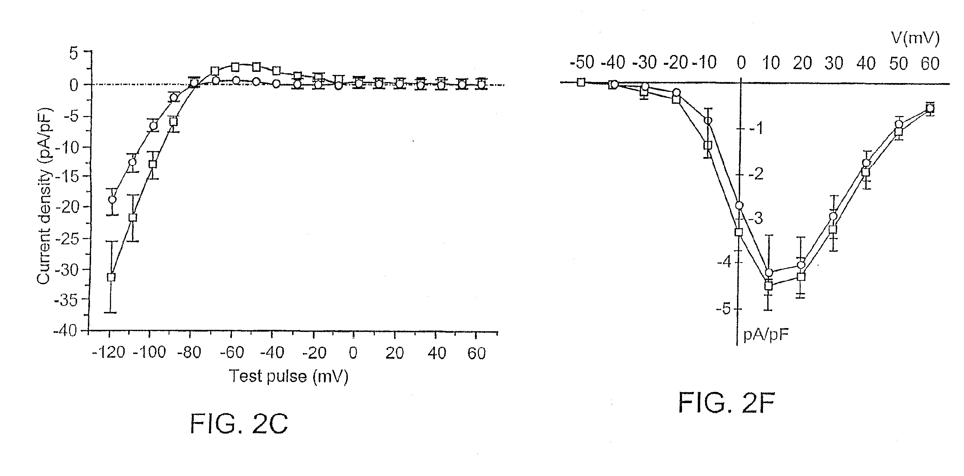

Figure 2. Specificity of $I_{K1}$ suppression. (A,B,C) The average current density of $I_{K1}$ was significantly reduced in Kir2.1AAA-transduced cells (n=9) compared with control cells (n=7, P<0.0001). (D,E,F). Further showing that the results are primarily due to the specific effects of modulating functional Kir2.1 channel number, the L- type calcium current was not altered in Kir2.1-AAA transduced myocytes (-4.2$\pm$0.9 pA/pF, n=4) compared to nontransduced cells (-4.5$\pm$0.2 pA/pF, n=6).

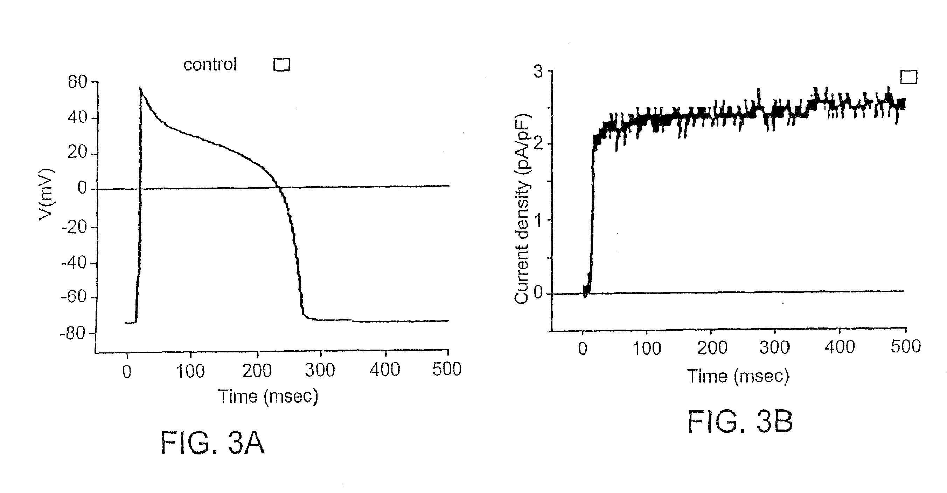

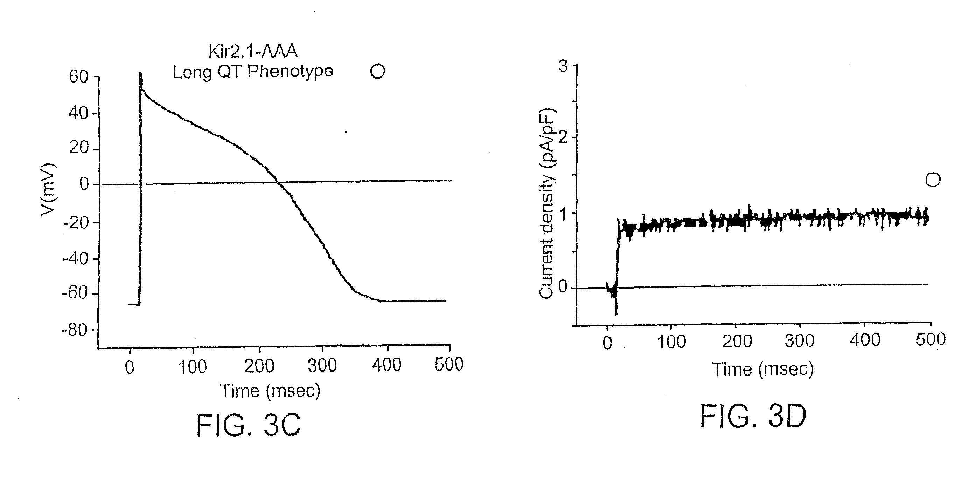

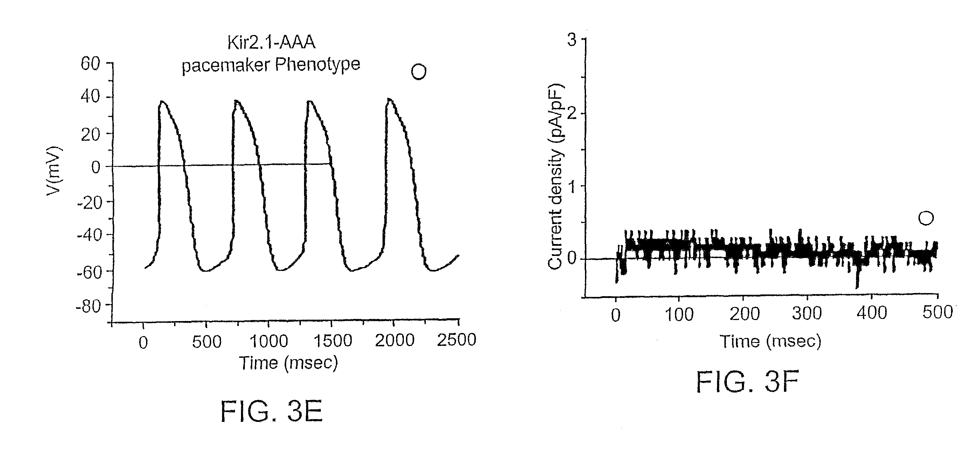

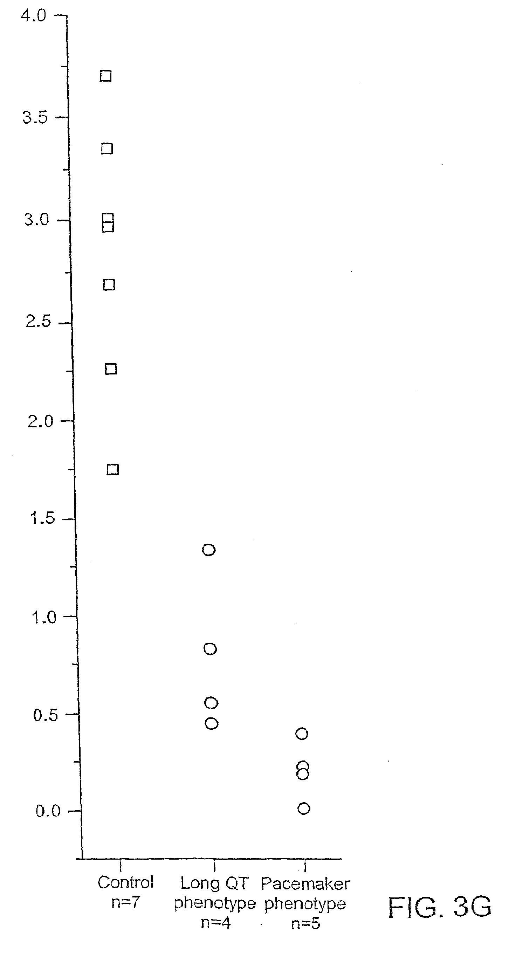

Figure 3. Action potential phenotype is determined by $I_{KI}$ density. (A) Stable APs are evoked by depolarizing external stimuli in control ventricular myocytes with a robust $I_{KI}$ (B, recorded at -50 mV). In Kir2.IAAA-transduced myocytes with moderately depressed $I_{KI}$ (D), APs with a long QT phenotype were evoked (C). Spontaneous APs (E) were observed in Kir2.1AAA cells with severely depressed $I_{K1}$ density (F). Three distinct ranges of $I_{K1}$ density (G) were recognized. Myocytes in which $I_{K1}$ was suppressed below 0.4 pA/pF exhibited a pacemaker phenotype.

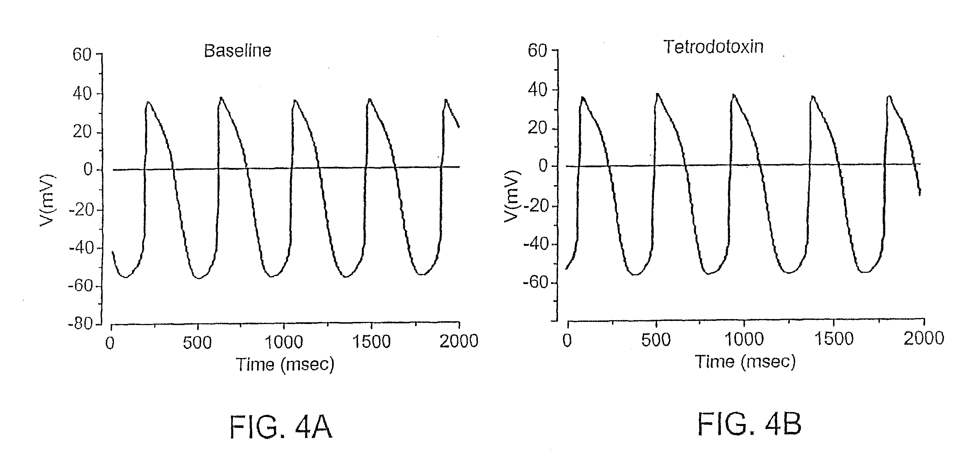

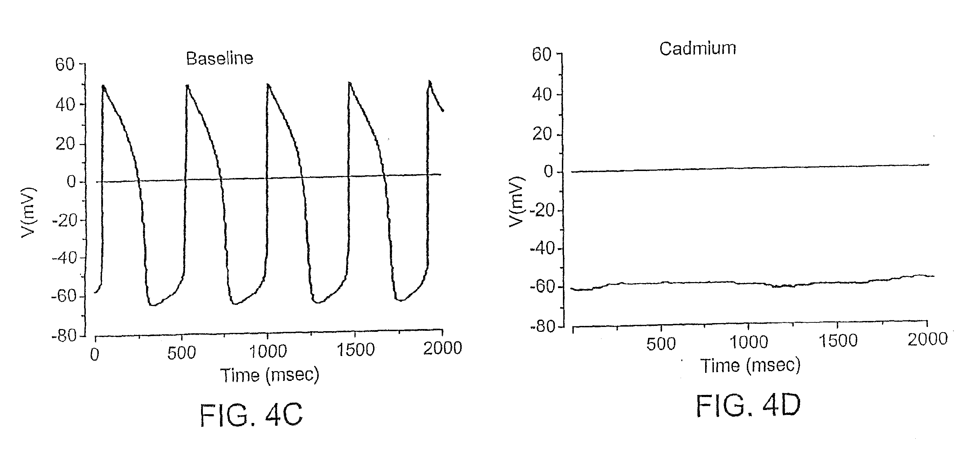

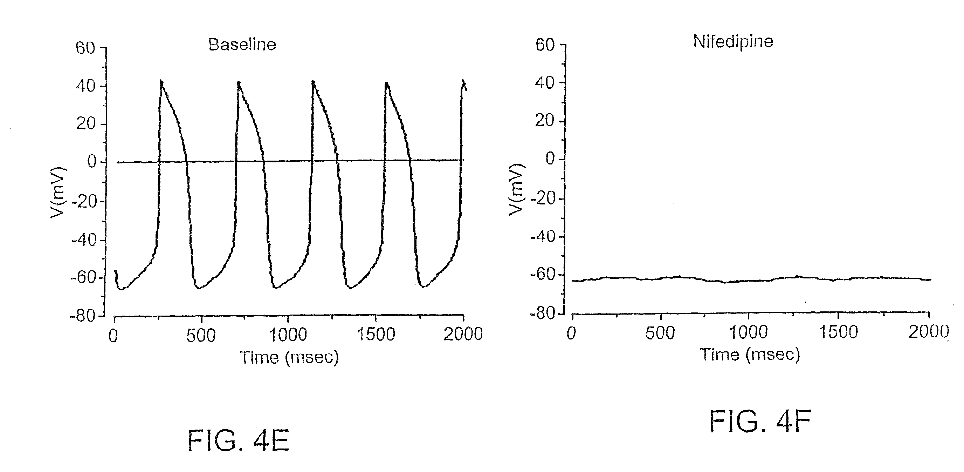

Figure 4. The calcium current is the excitatory current underlying the spontaneous APs. Kir2.1AAA-transduced cells with a pacemaker phenotype were unaffected by the Na channel blocker tetrodotoxin (10 $\mu$M, A,B), but spontaneous firing ceased during exposure to calcium channel blockers (cadmium 200 $\mu$M, C,D; nifedipine 10 $\mu$M, E,F).

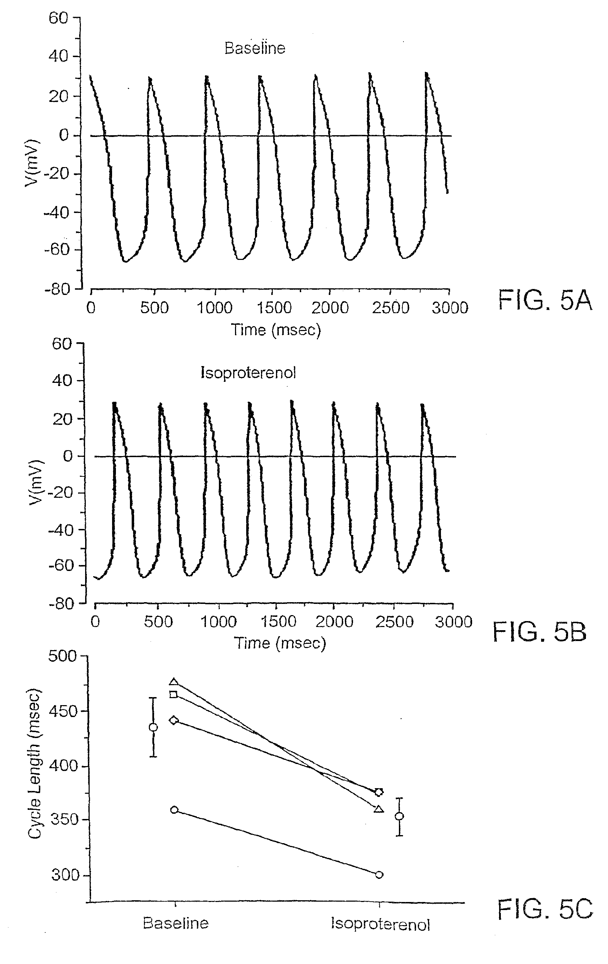

Figure 5. Application of isoproterenol (1 $\mu$M) increased the frequency of spontaneous AP in four Kir2.1AAA-transduced myocytes exhibiting pacemaking activity (A,B). Average cycle length was reduced from 435$\pm$27 ms at baseline to 351$\pm$18 ms (n=4) during isoproterenol exposure (P<0.01) (C).

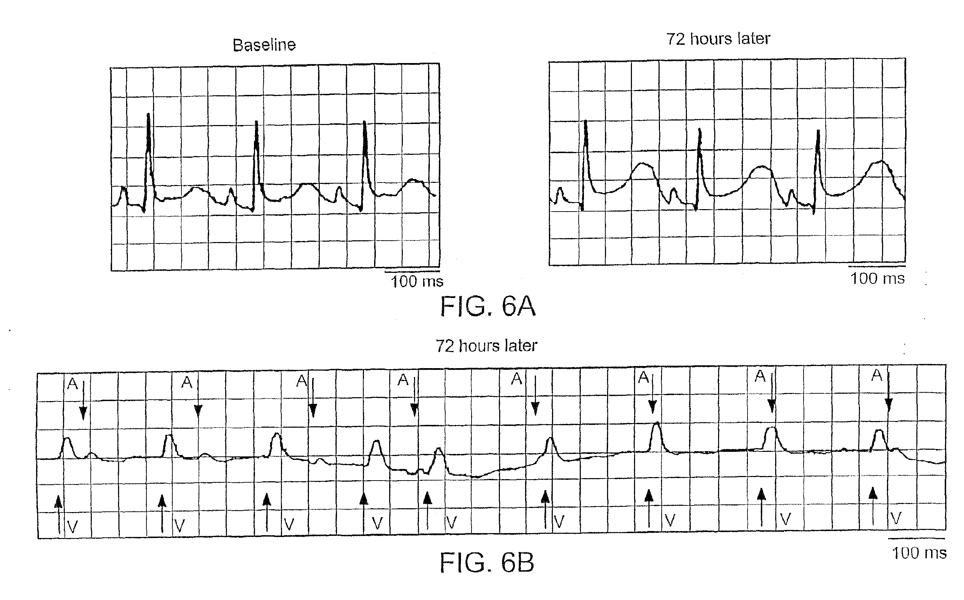

Figure 6. Electrocardiograms before and after gene delivery. (A) In 3 of 5 animals, QT intervals were prolonged 72 hours after gene transfer of Kir2.1AAA. (B) In 2 of 5 animals, ventricular rhythms developed. P waves (blue A and arrow) and wide QRS complexes (red V and arrow) march through to their own rhythm except a QRS complex inscribed with V which is a fusion beat. The baseline ECG recording for this animal was normal sinus rhythm (not shown, but similar to panel A).

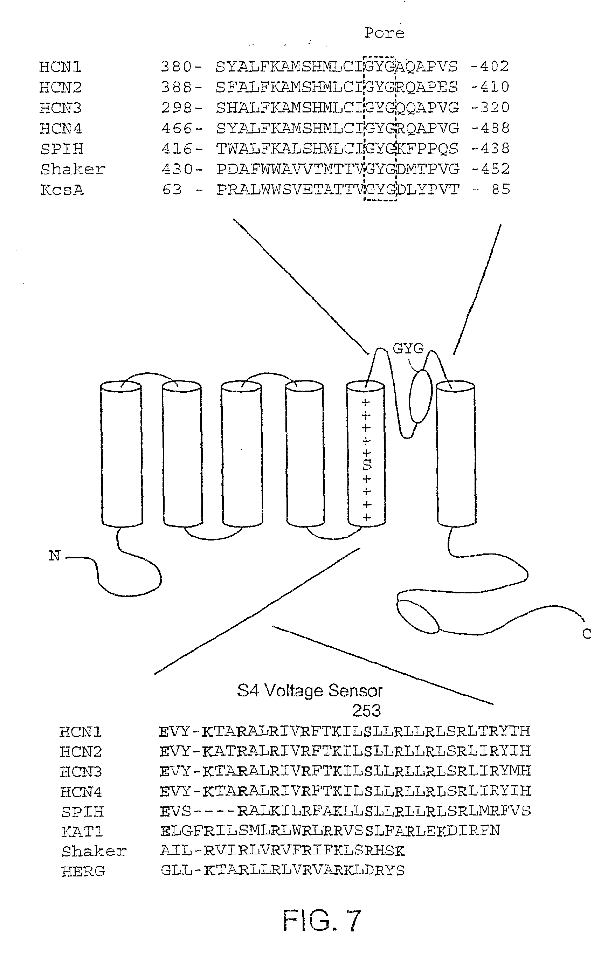

Figure 7 shows putative transmembrane topology of HCN-encoded pacemaker channels. In the middle panel: the six transmembrane segments (S1-S6) of a monomeric subunit of HCN1 channels are shown. The approximate location of the GYG signature motif is highlighted as shown. The cyclic nucleotide-binding domain (CNBD) is in the C-terminal region. In the top panel: sequence comparison of the ascending limb of the S5-S6 P-loops of various HCN and depolarization-activated (Kv) $K^+$ channels. The GYG triplet (highlighted) is conserved in all $K^+$-selective channels known except in rare occasions, such as that of the HERG $K^+$ channels, whose middle position is occupied by the conservative aromatic variant phenylalanine instead of tyrosine. The bottom panel compares the amino acid sequences of the S3-S4 linker and S4 segment of HCN isoforms 1-4 with those of a hyperpolarization-activated sea urchin sperm channel (SPH1), a hyperpolarization-activated $K^+$ channel cloned from the plant *Arabidopsis thaliana* (KAT1), and depolarization-activated *Shaker* and HERG $K^+$ channels. The S4 of HCN channels contains 9 basic amino acids regularly spaced from each other by two hydrophobic amino acids except at the sixth position, where a neutral serine is found in place of a cationic residue. SPIH and KAT1 channels have one fewer basic residue in their S4 segments compared to HCN channels, but again have a serine dividing the S4 into two portions. This S4 serine is not found in Kv channels; it divides the HCN voltage-sensing motif into two domains and has been hypothesized to be responsible for the unique hyperpolarization-activated opening of HCN channels.

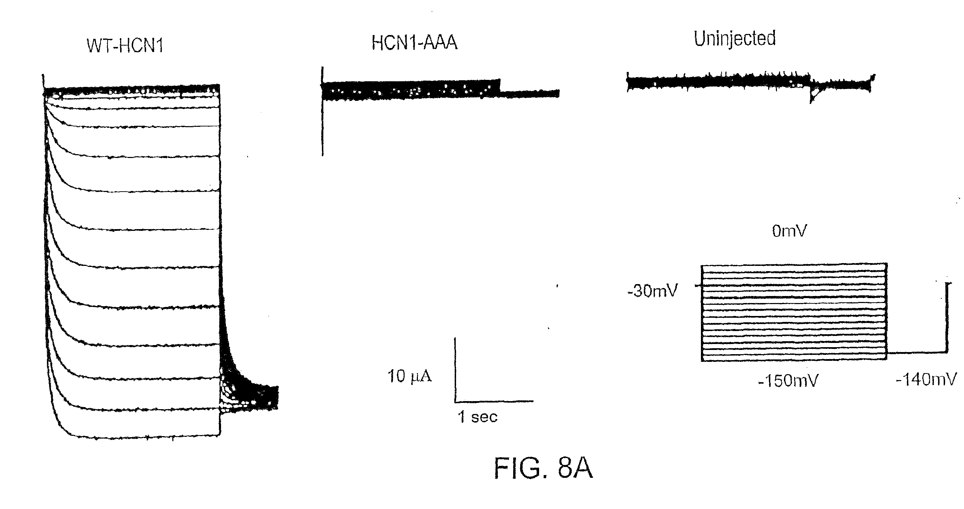

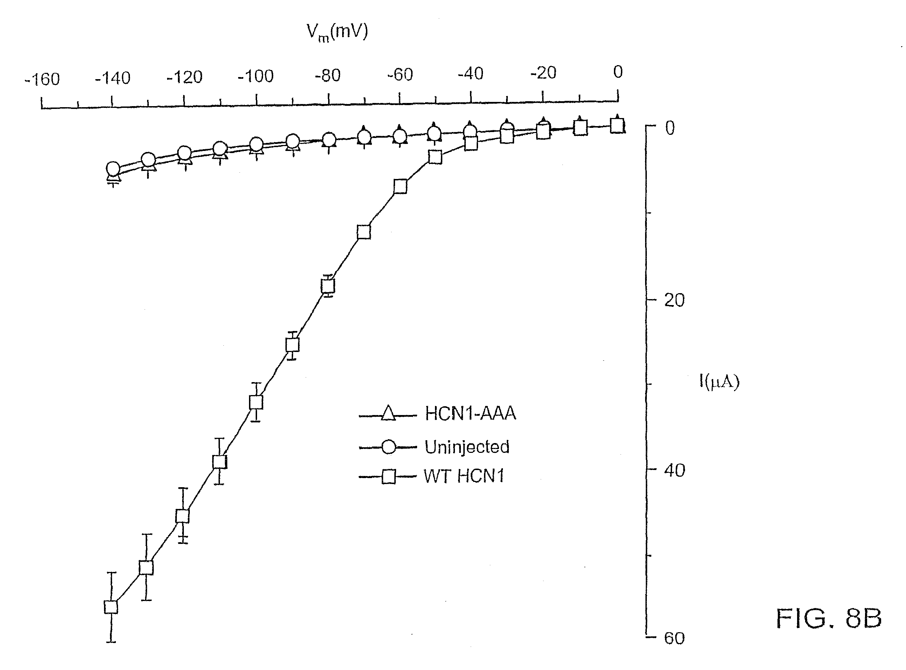

Figure 8 shows the effects of replacing GYG triplet in HCN1 with alanines (GYG$_{365-367}$AAA) on HCN1 currents. A) Representative traces of whole-cell currents recorded from oocytes injected with WTHCN1 and HCN1-AAA cRNA, and an uninjected oocyte as indicated. The electrophysiological protocol used to elicit currents is given in the inset. A family of 3-sec electrical pulses ranging from 0 to -150 mV in 10 mV increments was applied to oocytes from a holding potential of -30 mV. Tail currents were recorded at -140 mV. Whereas hyperpolarization-activated time-dependent currents were obvious from oocytes injected with WT HCN1, no measurable currents were observed from HCN1-AAA-injected and uninjected cells when the same protocol was used. B) Steady-state current-voltage relationships of WT HCN1- and BCN1-AAA-injected (solid squares and triangles, respectively), and uninjected (open circles) oocytes- Data shown are mean $\pm$ SEM.

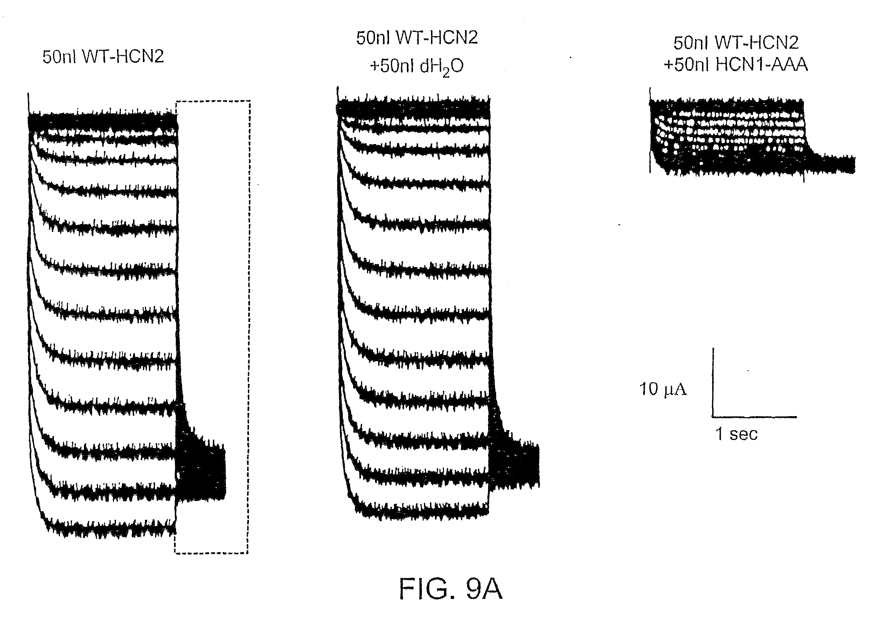

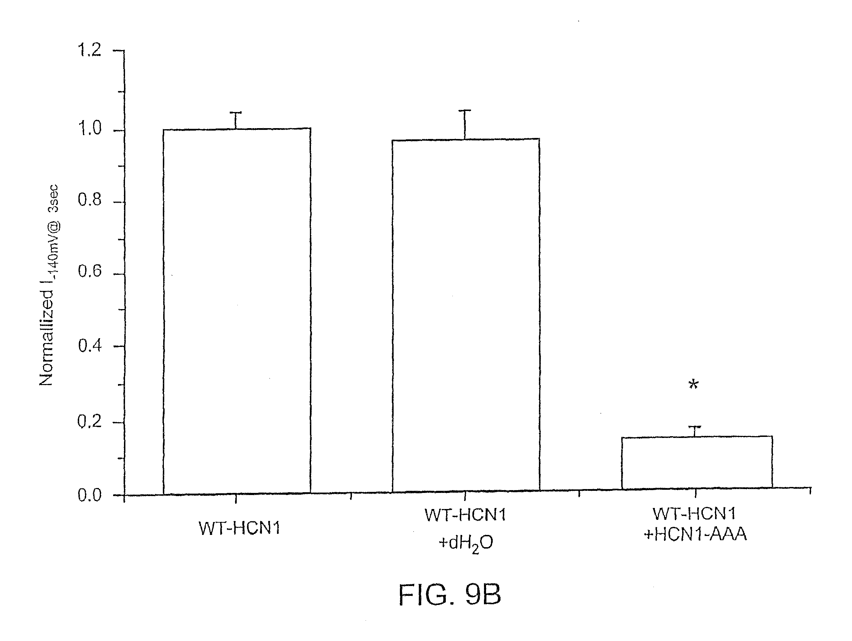

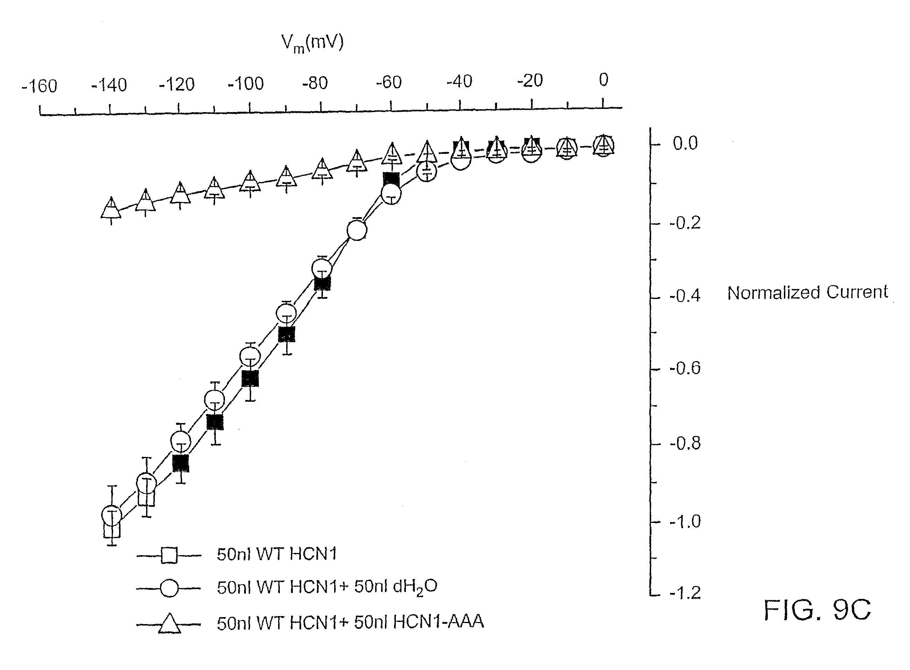

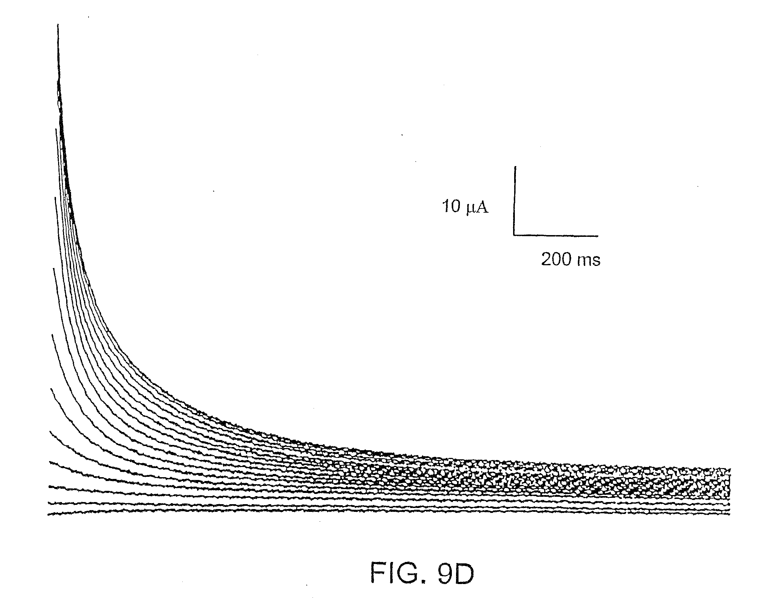

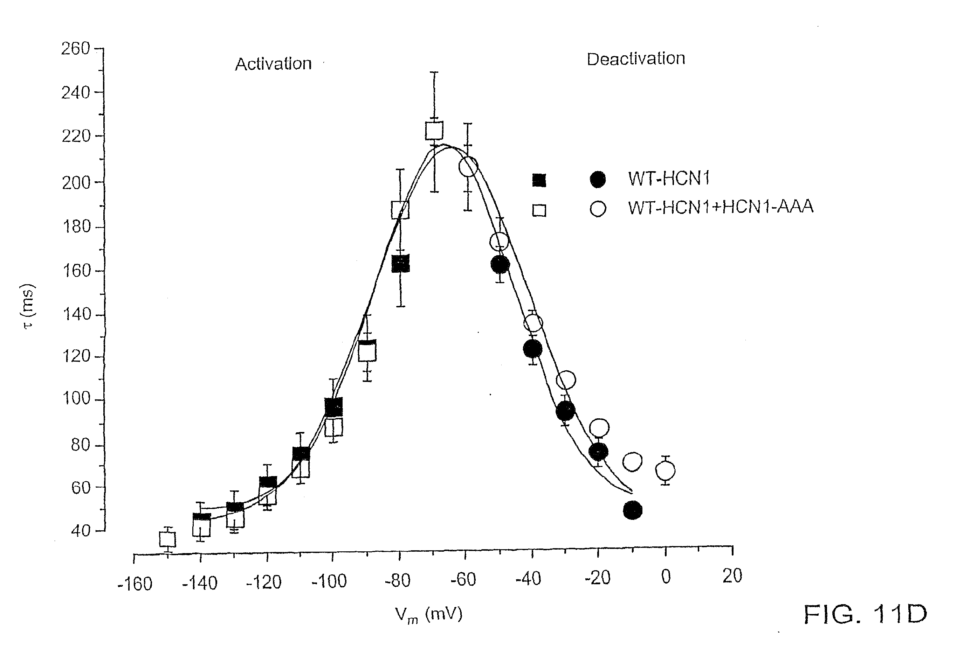

Figure 9 shows HCN1 AAA suppressed the normal activity of WT HCN1 in a dominant-negative manner. A) Representative current tracings recorded from oocytes injected with 50 nL WT HCN1, 50 nL WT HCN1+5 nL dH$_2$0, and 50 nL WT HCN1+50 nL HCN1-AAA cRNA (concentration = 1 ng/nL). The same voltage protocol from Figure 8 was used. Co-injection of WT HCN1 and HCN1-AAA significantly suppressed normal channel activity. WT HCN1 tail currents (enclosed in a box) are magnified in D). B) Bar graph summarizing the averaged current magnitudes of each of the groups from A) measured at the end of a 3 second pulse to - 140 mV from a holding potential of- 30 mV normalized to that of 50 nL WT HCN alone. *, p<0.01. C) Steady-state current-voltage relationships of the same groups from A). D) Tail currents of WT HCN1 at -140 mV. Fitting these currents with a mono-exponential function allows estimation of the time constants for activation (cf. Figure 11D).

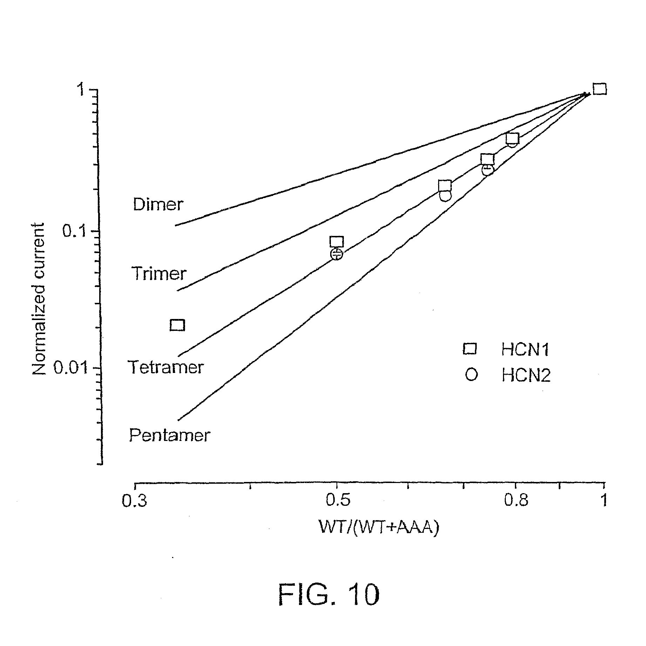

Figure 10 shows dominant-negative effect of HCN1-AAA on WT-HCN1, and 2 with varied WT:AAA ratio. Current suppression of WT HCN1 and HCN2 by HCN1-AAA plotted against the WT:AAA ratio of cRNA injected. Suppression of both HCN1 and HCN2 increased with decreasing WT:AAA ratio. Broken lines represent the suppression-ratio relationship statistically predicted from dimerization, trimerization, tetramerization and pentamerization of HCN monomers as indicated. The data also indicates that the endogenous HCN channel activity can be modulated by the AAA contruct disclosed herein.

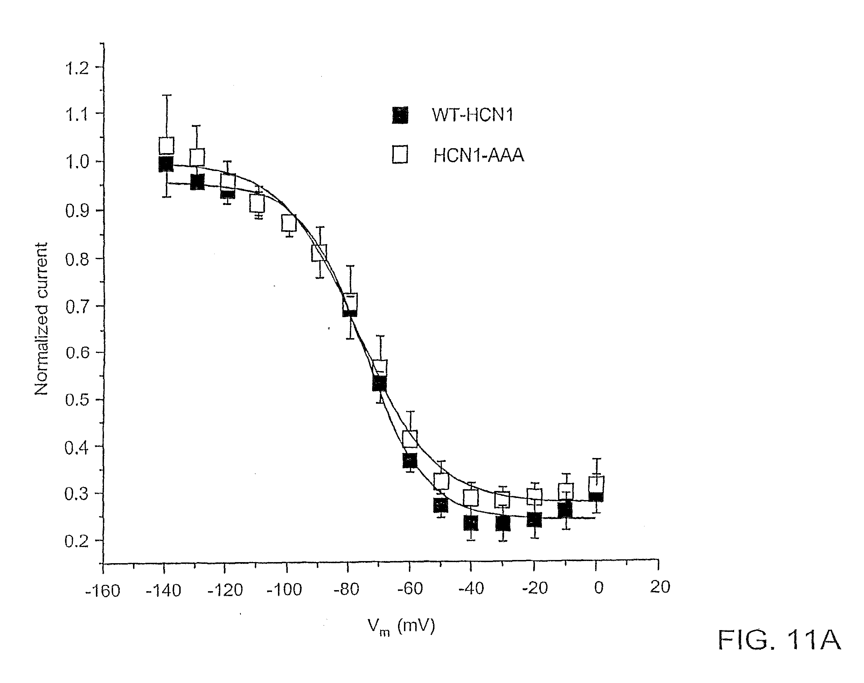

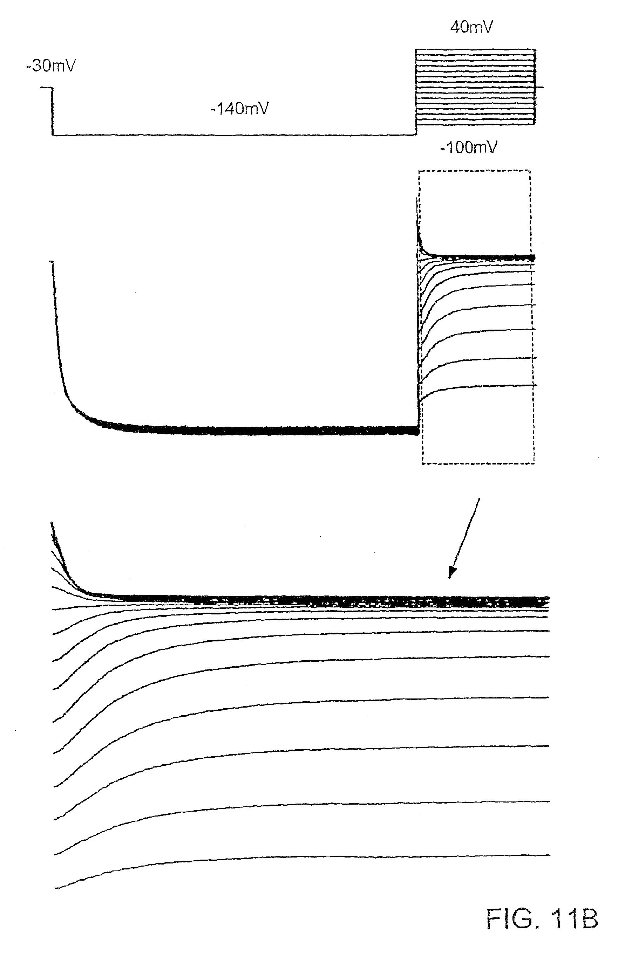

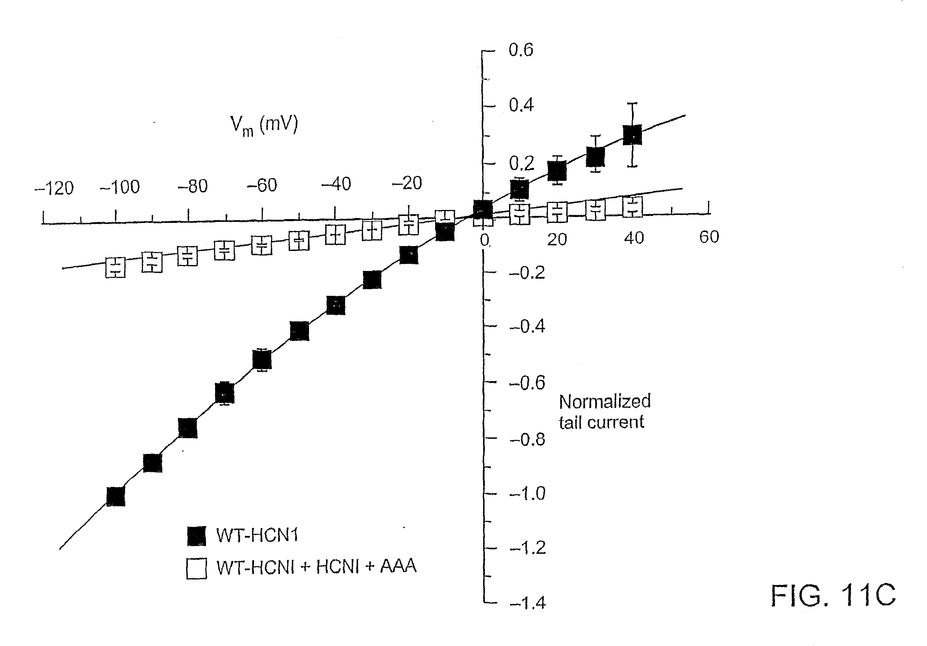

Figure 11 shows dominant-negative suppressive effect of HCN1-AAA did not alter gating and permeation properties of HCN1 channels.

A) Steady-state activation curves of WT HCN1 alone and after suppression by HCN1AAA (ratio= 1:1). Tail currents were measured immediately after pulsing to -140 mV using the same protocol as Figure 9A (cf, inset), normalized to the largest tail recorded and plotted against the preceding prepulse potentials. Neither the midpoint nor the slope factor was different among the two groups.

B) Electrophysiological protocol used for obtaining tail current-voltage relationships by stepping membrane potentials from -100 to +40 mV with 10 mV increments after a 3 second prepulse to-140 mV. A representative family of tail currents recorded from an oocyte injected with 50 nL of 1 ng/nL WT HCN1 cRNA only is shown, and magnified as shown. Fitting these currents with a mono-exponential function allows estimation of the time constants for deactivation ($\tau_{deact}$).

C) Tail current-voltage relationships measured from oocytes injected with WT HCN1 alone or co-injected with WT HCN1 and HCN1-AAA (ratio=1:1). Whole-cell currents were suppressed by HCN1-AAA but the reversal potential was not changed. D) Summary of $\tau_{act}$ (squares) and $\tau_{deact}$ (circles) of currents induced by the injection of WT HCN1 alone (solid symbols) or by 1:1 co-injection of both WT HCN1 and HCN1-AAA (open symbols). Distribution of i was bell-shaped with midpoints similar to those derived from the corresponding steady-state activation curves. Gating kinetics of expressed currents were also not changed by HCN1-AAA co-injection.

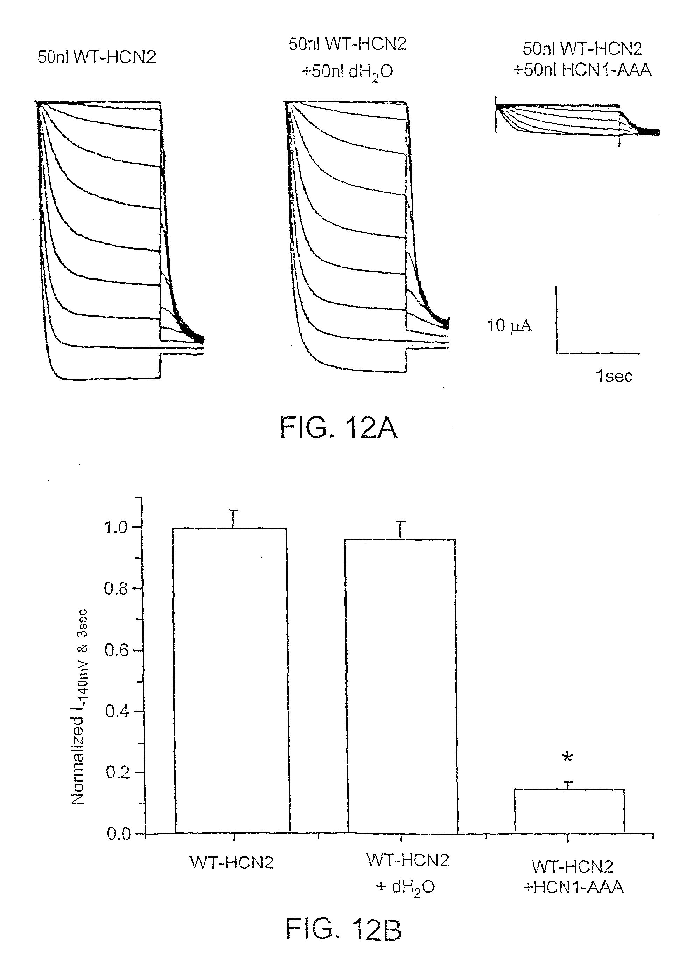

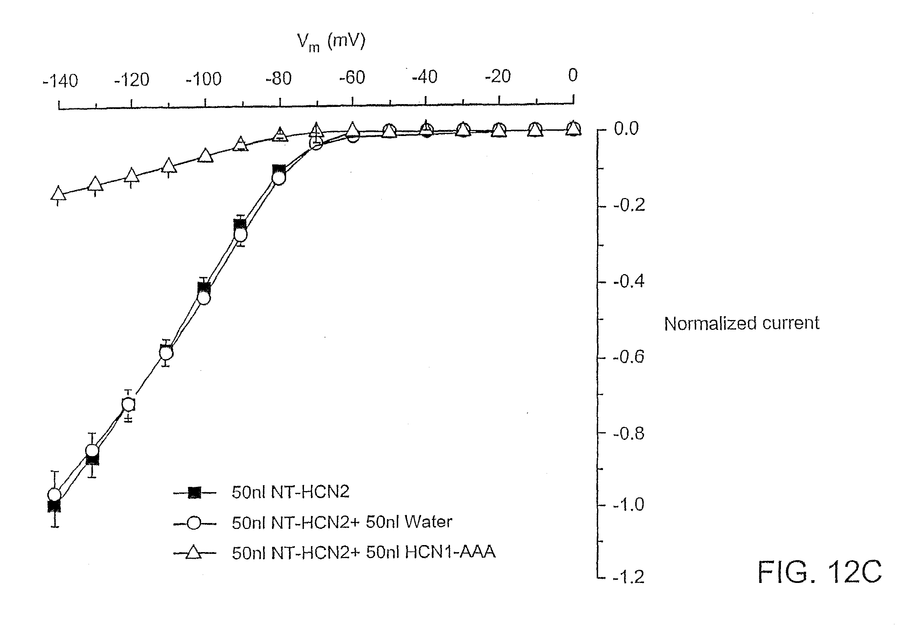

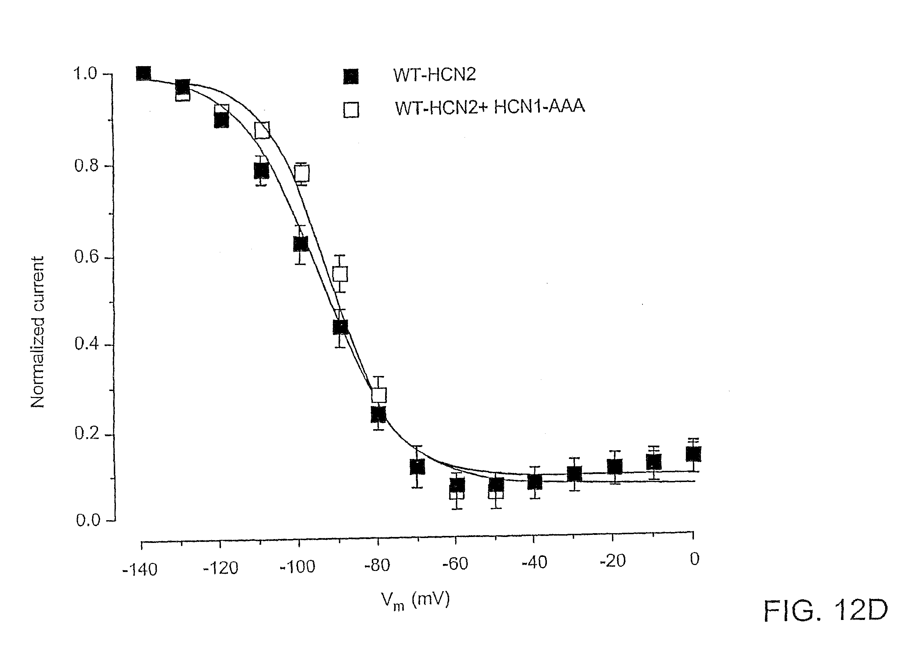

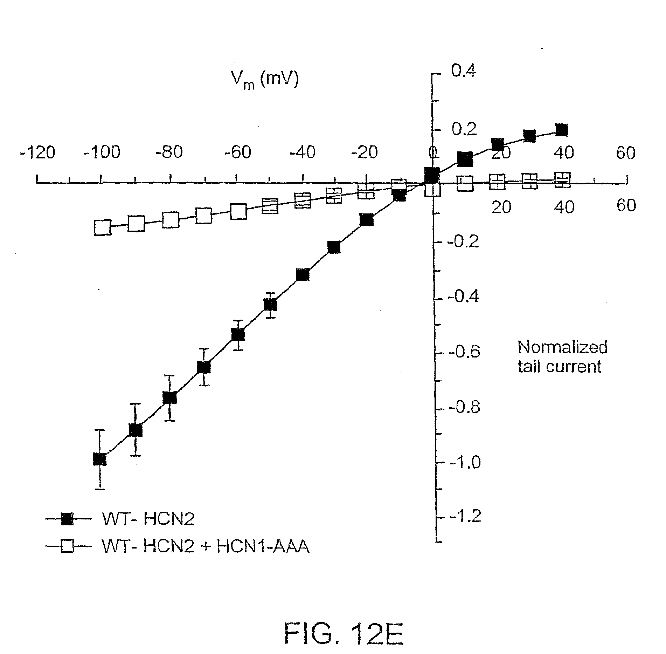

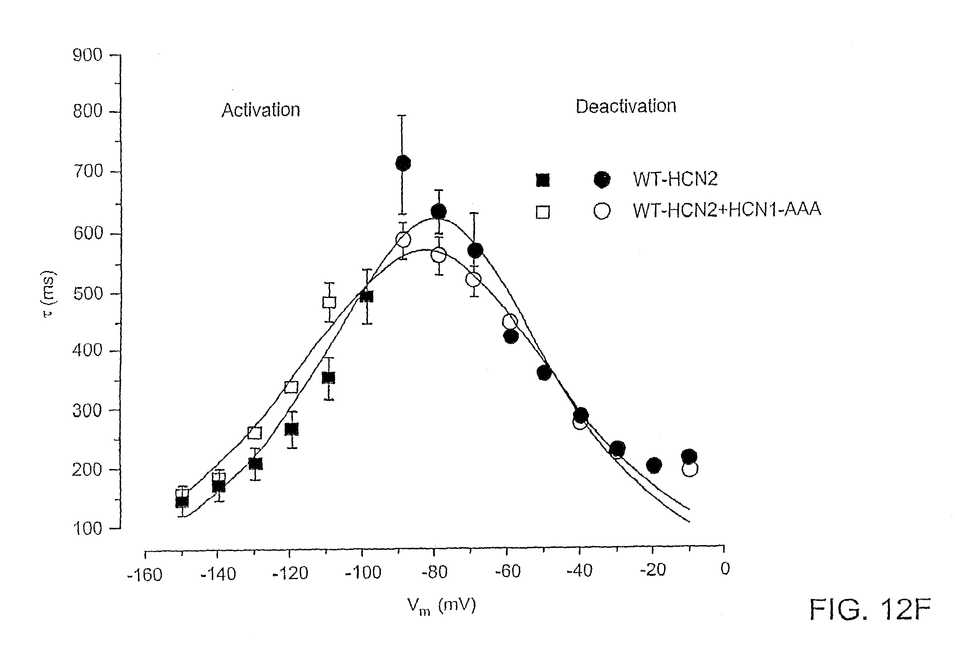

Figure 12 shows effects of HCN1-AAA on HCN2 channels.

(A) Representative current tracings recorded from oocytes injected with 50 nL WT HCN2, 50 nL WT HCN2+50 nL dH$_2$0, and 50 nL WT HCN2+50 nL HCN1-AAA cRNA. HCN1-AAA also suppressed the activity of WT HCN2. (B) Current suppression at -140 mV of WT HCN2 by HCN1-AAA plotted against the WT HCN2:HCN1-AAA ratio of cRNA injected. C. Steady-state current-voltage relationships of the same groups from A). Steady-state activation (D), reversal potential (E), and activation and deactivation kinetics (F) of WT HCN2 expressed alone and co-expression with HCN1-AAA (ratio=1:1) were identical (p>0.05).

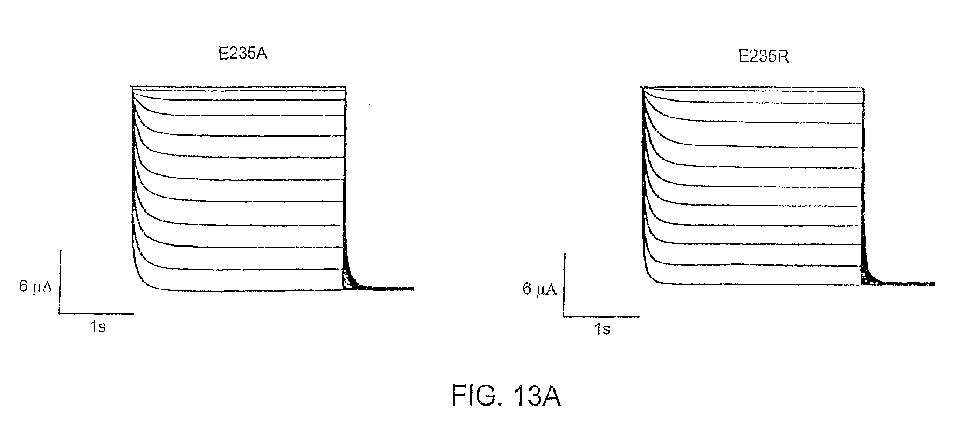

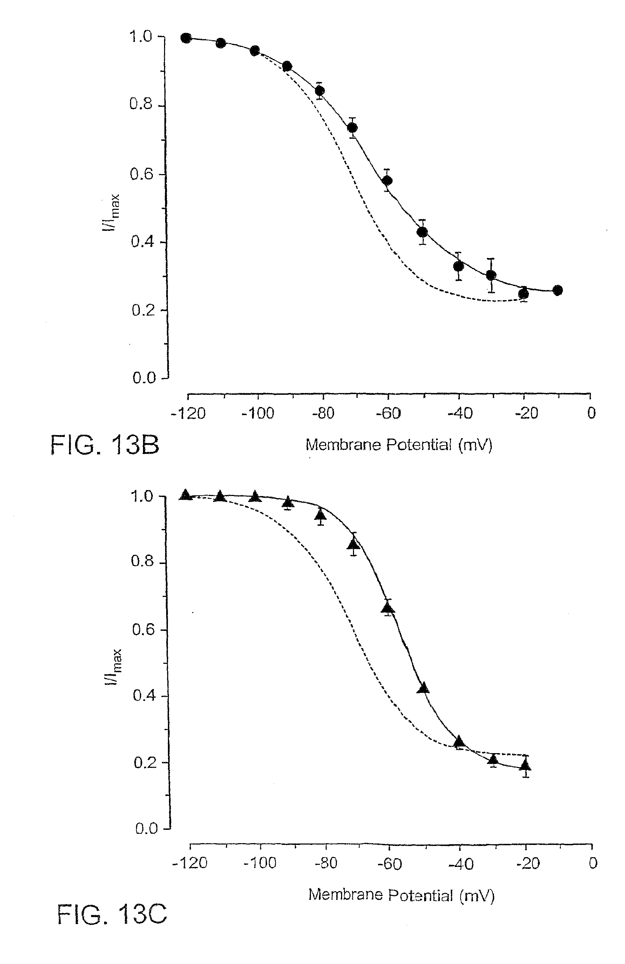

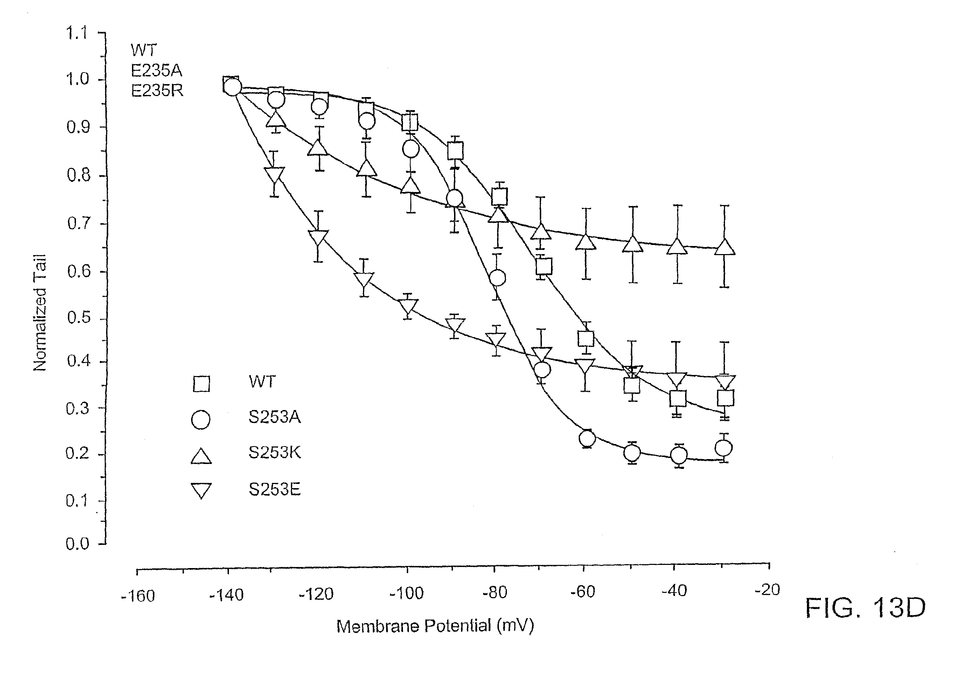

Figure 13 shows the effects of E235 mutations on HCN1 activation gating. A) Representative records of currents through E235A and E235R HCN1 channels elicited using the voltage protocol in Figure 8. B) Steady-state activation curve of WT and E235A. The activation curve for E235A is shifted positively. C) Steady-state activation curve of WT and E235R. The activation curve for E235R is shifted even more positively than that of E235A, showing a greater effect with a net charge change of +2 as compared to +1. D) Steady-state activation curves of WT, S253A, S253K and S253E channels. The conservative S-to-A mutant shows a shift of activation but has a preserved slope factor and $P_{o,min}$. Despite the opposite charges of these substitutions, the activation curves for both S253K and S253E are shifted far negatively. Taken collectively, this shows that the activation threshold of HCN channel activity (Figure 13) can be modulated as well as the endogenous expressed current amplitude (Figures 8-12).

DETAILED DESCRIPTION OF THE INVENTION

As discussed above, we now provide gene transfer and cell administration methods to induce and/or modulate the activity of an endogenous or induced cardiac pacemaker function. In particular, the invention provides for the creation of genetically-engineered pacemakers using gene therapy as an alternative and/or supplement to implantable electronic pacemakers. In preferred aspects of the invention, quiescent heart muscle cells are converted into pacemaker cells by in vivo viral gene transfer. Cardiac contraction and/or an electrical property of those converted cells then may be modulated in accordance with the invention.

More particularly, in a first aspect of the invention, methods of the invention may be employed to induce a pacemaker function (cardiac contraction) in myocardial cells that have not been exhibiting such properties, i.e. quiescent myocardial cells that exhibit no, little or inappropriate firing rate. Preferably, the administration induces or otherwise causes the treated cardiac cells to generate spontaneous repetitive electrical signals, i.e. for myocardial cells that exhibited little (firing rate of about 20, 15, 10, 5 per minute or less) or no firing rate, the frequency of the firing rate or electrical signal output will preferably increase to a detectable level, particularly a firing rate or electrical signal output increase of at least about 3, 5, 10, 15, 20 or 25 percent after the administration.

In a further aspect, methods of the invention are employed to modulate or "tune" the existing firing rate of myocardial cells. In this aspect, excessive ventricular pacing may be decreased to a decreased frequency or firing rate, or ventricular pacing rates that are too low may be increased to a desired level. This aspect of the invention is particulary useful to modulate the effect achieved with an implanted (electronic) pacemaker effect to provide an optimal heart rate for a patient. Further, the invention has the advantage of maintaining the responsiveness of tissues being treated to endogenous neuronal or hormonal inputs.

Significantly, the invention may be employed to augment or supplement the effect of an implanted electronic pacemaker. That is, a mammal that has an implanted electronic pacemaker may be treated in accordance with the invention, i.e. a composition such as a polynucleotide or modified cells may be administered to the mammal to further modulate cardiac firing rate that is provided by the implanted electronic device. By such a combined approach, a precise and optimal firing rate can be achieved. Additionally, the composition can be administered to a site in the mammalian heart that is remote from the electronic pacemaker, e.g. the composition administration site being at least about 0.5, 1, 2, 3, 4 or 5 centimeters from the implanted electronic device, to thereby provide a pacemaker effect through a greater area of cardiac tissue.

Methods of the invention may suitably be employed to modulate a treated subject's cardiac firing rate to within about 15 or 10 percent of a desired firing rate, more preferably about 8, 5, 4, 3 or 2 percent of a desired firing rate value.

Preferred methods of the invention include administration of a polynucleotide that codes for, particularly a polynucleotide that is introduced into pacemaker cells such as in the sinoatrial node of a mammalian heart, or administration of inducible cells such as pacemaker cells created from stem cells or converted from electrically quiescent cells, or other cells adapted to generate rhythmic contraction or cardiologic excitation. As discussed above, preferred methods involve administering a therapeutically effective amount of at least one polynucleotide or modified cell capable of modulating heart contraction (firing rate). Polynucleotides and modified cells also are preferred therapeutic compositions for administration in accordance with the invention due to the ease of localized administration of those agents within a targeted region of cardiac tissue.

Suitable compositions for administration to modulate firing rate of myocardial cells also can be readily identified by simple testing, i.e. a candidate agent such as a polynucleotide can be administered to myocardial cells to determine if the administered agent modulates firing rate relate to control myocardial cells (same cells that are untreated with agent) as determined for instance by a standard electrophysiological assay as such assay is defined below.

Particularly preferred polynucleotides for administration are dominant-negative constructs. These constructs, include but are not limited to, for example, Kir2 constructs, HCN constructs, mutants, fragments and combinations thereof.

In a preferred embodiment of the invention, somatic gene transfer of a dominant-negative Kir2 constructs produces spontaneous pacemaker activity in the ventricle, resulting in the creation of biological pacemakers by localized genetic suppression of $I_{K1}$ of dormant pacemakers present within the working myocardium.

For instance, a Kir2 dominant-negative construct can be produced by, for example, replacement of amino acid residues in the pore region of Kir2.1 by alanines ($GYG_{144\text{-}J46} \rightarrow AAA$, or Kir2.1AAA). Such a dominant-negative construct can suppress current flux when co-expressed with wild-type Kir2.1. Incorporation of at least about one single mutant subunit within the tetrameric Kir channel can be sufficient to knock out function. The dominant-negative construct, for example, Kir2.1AAA can be packaged into a bicistronic vector and injected into the left ventricular cavity of guinea pigs. Preferably, in a localized area such as an area of approximately 1 cubic cm, at least about 10% of ventricular myocytes are transduced, more preferably at least about 20% ventricular myocytes are transduced, most preferably at least about 30%, 40% or 50% ventricular myocytes are transduced. Measurement of $I_{K1}$ and calcium currents are conducted as described in detail in the examples which follow.

The above activities of transduced myocytes are compared to control ventricular myocytes spontaneous activity as described in the Examples which follow. It is desirable for the transduced myocytes to exhibit spontaneous activity representative of pacemaker cells, such as the early embryonic heart cells which possess intrinsic pacemaker activity or the normal pacemaker cells of the sinoatrial node.

In another preferred embodiment, the invention provides for antisense therapeutic molecules which inhibit the expression of Kir2 gene products. In therapeutic applications oligonucleotides have been used successfully to block translation in vivo of specific mRNAs thereby preventing the synthesis of proteins which are undesired or harmful to the cell/organism. This concept of oligonucleotide mediated blocking of translation is known as the "antisense" approach. Mechanistically, the hybridizing oligonucleotide is thought to elicit its effect by either creating a physical block to the translation process or by recruiting cellular enzymes that specifically degrade the mRNA part of the duplex (RNaseH).

To be useful in an extensive range of applications, oligonucleotides preferably satisfy a number of different requirements. In antisense therapeutics, for instance, a useful oligonucleotide must be able to penetrate the cell membrane, have good resistance to extra- and intracellular nucleases and preferably have the ability to recruit endogenous enzymes like RNaseH. In DNA-based diagnostics and molecular biology other properties are important such as, e.g., the ability of oligonucleotides to act as efficient substrates for a wide range of different enzymes evolved to act on natural nucleic acids, such as *e.g.* polymerases, kinases, ligases and phosphatases. Oligonucleotides used as antisense therapeutic molecules need to have both high affinity for its target RNA to efficiently impair its translation and high specificity to avoid the unintentional blocking of the expression of other proteins.

In particular, it is preferred to have antisense oligonucleotides which inhibit the expression of at least about one component that makes up the Kir2 channel, or enough components that make up the Kir2 channels, to specifically suppress Kir2 channels sufficient to unleash pacemaker activity in ventricularmyocytes, as measured by the partial suppression or the absence of strongly-polarizing $I_{K1}$.

Other administration protocols may be employed. For example, the administered polynucleotide may function as a decoy, where the polynucleotide is introduced to targeted cells or genes by any convenient means, wherein activation of a gene is interrupted e.g. by diverting transcription factors to the decoy molecule. More particularly, a decoy can be employed to effectively inhibit expression of a Kir2 channel component. As used herein, the term "decoy molecule" or other similar term includes reference to a polynucleotide that codes for a functionally inactive protein or the protein itself which competes with a functionally active protein and thereby inhibits the activity promoted by the active protein. A Kir2 decoy protein can thus acts as a competitive inhibitor to wild type Kir2 proteins thereby inhibiting formation of Kir2 wild type channels and resulting in suppression of inward rectifier potassium current ($I_{K1}$).

Preferably, the dominant negative constructs must be durable, i.e. long-lasting, such as for months for years and regionally specific, i.e. only target the desired tissue and specifically act on the mechanism of choice, for example, a Kir2 dominant-negative construct specifically suppresses Kir2 channels sufficient to unleash pacemaker activity in ventricular myocytes, as measured by the absence of strongly-polarizing $I_{K1}$.

Another example of a dominant-negative construct for use in accordance with the invention is an HCN construct, such as an HCN1 construct. Particularly, in such a construct, the critical residues GYG in the pore have been converted to AAA (to create HCN1-AAA), that is capable of suppressing the normal HCN-encoded pacemaker currents.

More particularly, as further shown in the examples which follow, the functional importance of the GYG selectivity motif in pacemaker channels was evaluated by replacing that triplet in HCN1 with alanines ($GYG_{365-367}AAA$). HCN1-AAA did not yield functional currents; co-expression of HCN1-AAA with WT HCN1 suppressed normal channel activity in a dominant-negative manner ($55.2 \pm 3.2$, $68.3 \pm 4.3$, $78.7 \pm 1.6$, $91.7 \pm 0.8$, $97.9 \pm 0.2\%$ current reduction at -140 mV for WT:AAA ratios of 4:1, 3:1, 2:1, 1:1 and 1:2, respectively) without affecting gating (steady-state activation, activation and deactivation kinetics) or permeation (reversal potential) properties. Statistical analysis reveals that a single HCN channel is composed of four monomeric subunits. Interestingly, HCN1-AAA also inhibited HCN2 in a dominant-negative manner with the same efficacy- It is thus believed that the GYG motif is a critical determinant of ion permeation for HCN channels, and that HCN1 and HCN2 readily coassemble to form heterotetrameric complexes.

As indicated above, through the co-assembly of different HCN isoforms, endogenous HCN activity (e.g. activation thresholds and expressed current amplitudes) can be modulated in both directions thereby enabling effective modulation of cardiac pacing or firing rate, such as within a preferred range of a desired value as discussed above.

The invention is generally compatible with one or a combination of suitable polynucleotide administration routes including those intended for *in* vivo or ex vivo cardiac use. There is understanding in the field that cardiac tissue is especially amenable to gene transfer techniques. See e.g, Donahue, J. et al. (1998) Gene Therapy 5: 630; Donahue, J. et al. PNAS (USA) 94: 4664 (disclosing rapid and efficient gene transfer to the heart); Akhttr, S. et al. (1997) PNAS (USA) 94: 12100 (showing successful gene transfer to cardiac ventricular myocytes); and references cited therein. Preferred nucleic acid delivery methods are disclosed in U.S. Patent 6,376,471.

Further preferred administration routes according to the invention involve introducing the polynucleotide into cardiac tissue and expressing same sufficient to detectably decrease heart rate as determined by a standard electrocardiogram (ECG) recording. Preferably, the decrease in heart rate is at least about 5% relative to baseline.

The invention is highly flexible and can be used with one or a combination of polynucleotides, preferably those encoding at least one therapeutic heart protein.

In addition to the preferred polynucleotides discussed above, suitable polynucleotides for administration in accordance with the invention include, but are not limited to, those encoding at least one ion channel protein, gap junction protein, G protein subunit, connexin; or functional fragment thereof More preferred are poly-nucleotides encoding a K channel subunit, Na channel subunit, Ca channel subunit, an inhibitory G protein subunit; or a functional fragment thereof. Additionally preferred polynucleotides will encode one, two or three of such proteins (the same or different).

By the phrase "fragment", "function fragment" or similar term is meant a portion of an amino acid sequence (or polynucleotide encoding that sequence) that has at least about 70%, preferably at least about 80%, more preferably at least about 95% of the function of the corresponding full-length amino acid sequence (or polynucleotide encoding that sequence). Methods of detecting and quantifying functionality in such fragments are known and include the standard electrophysiological assays disclosed herein.

Suitable polynucleotides for practicing the invention can be obtained from a variety of public sources including, but not limited to, GenBank (National Center for Biotechnology Information (NCBI)), EMBL data library, SWISS-PROT (University of Geneva, Switzerland), the PIR-International database; and the American Type Culture Collection (ATCC) (10801 University Boulevard, Manassas, VA 20110-2209). See generally Benson, D.A. et al. (1997) Nucl. Acids. Res. 25: 1 for a description of Genbank.

More particular polynucleotides for use with the present invention are readily obtained by accessing public information from GenBank. For example, in one approach, a desired polynucleotide sequence is obtained from GenBank. The polynucleotide itself can be made by one or a combination of routine cloning procedures including those employing PCR-based amplification and cloning techniques. For example, preparation of oligonucleotide sequence, PCR amplification of appropriate libraries, preparation of plasmid DNA, DNA cleavage with restriction enzymes, ligation of DNA, introduction of DNA into a suitable host cell, culturing the cell, and isolation and purification of the cloned polynucleotide are known techniques. See e.g., Sambrook et al. in Molecular Cloning: A Laboratory Manual (2d ed. 1989); and Ausubel et al. (1989), Current Protocols in Molecular Biology, John Wiley & Sons, New York.

Table I below, references illustrative polynucleotides from the GenBank database for use with the present invention.

Table 1

| Polynucleotide | GenBank Accession No. |
| --- | --- |
| Kir 2.1 potassium channel | XM028411[1] |
| HERG potassium channel | XM004743 |
| Connexin 40 | AF151979 |
| Connexin 43 | AF151980 |
| Connexin 45 | U03493 |
| Na channel alpha subunit | NM000335 |
| Na channel beta-1 subunit | NM001037 |
| L-type Ca channel alpha-1 subunit | AF201304 |
| HCN1 | NM010408; |
| | AF247450; |
| | AF064876 |

[1] An additional polynucleotide for use with the present invention is the Kir2.1 AAA mutant, which is wild-type Kir 2.1 with a substitution mutation of AAA for GFG in position 144-146.

Additional polynucleotides for use with the invention have been reported in the following references: Wong et al. Nature 1991;351(6321):63 (constitutively active Gi2 alpha); ) De Jongh KS, et al. J Biol Chem 1990 Sep 5;265(25): 14738 (Na and Ca channel beta subunits); Perez-Reyes, E. et al. J Biol Chem 1992 Jan 25;267(3):1792; Neuroscientist 2001 Feb;7(1):42 (providing sodium channel beta subunit information); Isom, LL. Et al. Science 1992 May 8;256(5058): 839 (providing the beta I subunit of a brain sodium channel); and Isom, LL. Et al. (1995) Cell 1995 Nov 3;83(3):433 (reporting beta 2 subunit of brain sodium channels).

Further polynucleotides for use with the invention have been reported in PCT application number PCT/US98/23877 to Marban, E.

See also the following references authored by E. Marban: J. Gen Physiol. 2001 Aug; 118(2):171-82; Circ Res. 2001 Jul 20;89(2):160-7; Circ Res. 2001 Jul 20;89(2):101; Circ Res. 2001 Jul 6;89(1):33-8; Circ Res. 2001 Jun 22;88 (12):1267-75; J Biol Chem. 2001 Aug 10;276(32):30423-8; Circulation. 2001 May 22;103(20):2447-52; Circulation. 2001 May 15;103(19):23634; Am J Physiol Heart Circ Physiol. 2001 Jun;280(6):H2623-30; Biochemistry. 2001 May 22;40 (20):6002-8; J Physiol. 2001 May 15;533(Pt 1):127-33; Proc Nat1 Acad Sci U S A. 2001 Apr 24;98(9):5335-40; Circ Res. 2001 Mar 30;88(6):570-7; Am J Physiol Heart Circ Physiol. 2001 Apr;280(4):H1882-8; and J Mol Cell Cardiol. 2000 Nov;32(1 1): 1923-30.

Further examples of suitable Ca channel subunits include beta 1, or alpha2-delta subunit from an L-type Ca channel. A preferred Na channel subunit is betal or beta2. In some invention embodiments it will be useful to select Na and Ca channel subunits having dominant negative activity as determined by the standard electrophysiological assay described below. Preferably, that activity suppresses at least about 10% of the activity of the corresponding normal Na or Ca channel subunit as determined in the assay.

Particularly preferred constructs for administration in accordance with the invention also are disclosed in the examples which follow.

Also preferred is the inhibitory G protein subunit ("$G\alpha_{i2}$") or a functional fragment thereof, as a supplemental strategy to modulate pacemaker activity.

The invention is broadly suited for use with gap junction proteins, especially those known or suspected to be involved with cardiac function. Particular examples include connexin 40,43, 45; as well as functional fragments thereof. Further contemplated are polynucleotides that encode a connexin having dominant negative activity as determined by the assay, preferably a suppression activity of at least about 10% with respect to the corresponding normal connexin 40, 43, or 45. Connexins may be particularly useful to induce/force stem cells or derived cardiomyocytes to form electrical couplings with quiescent heart tissue.

Also envisioned are mutations of such polynucleotides that encode dominant negative proteins (muteins) that have detectable suppressor activity. Encoded proteins that are genetically dominant typically inhibit function of other proteins particularly those proteins capable of forming binding complexes with the wild-type protein.

Additional polynucleotides of the invention encode essentially but not entirely full-length protein. That is, the protein may not have all the components of a full-length sequence. For example, the encoded protein may include a complete or nearly complete coding sequence (cds) but lack a complete signal or poly-adenylation sequence. It is preferred that a polynucleotide and particularly a cDNA encoding a protein of the invention include at least a complete cds. That cds is preferably capable of encoding a protein exhibiting a molecular weight of between about 0.5 to 70, preferably between about 5 and 60, and more preferably about 15, 20, 25, 30, 35, 40 or 50 kD. That molecular weight can be readily determined by suitable computer-assisted programs or by SDS-PAGE gel electrophoresis.

The polynucleotide and particularly the cDNA encoding the full-length protein can be modified by conventional recombinant approaches to modulate expression of that protein in the selected cells, tissues or organs.

More specifically, suitable polynucleotides can be modified by recombinant methods that can add, substitute or delete one or more contiguous or non-contiguous amino acids from that encoded protein. In general, the type of modification conducted will relate to the result of expression desired.

For example, a cDNA polynucleotide encoding a protein of interest such as an ion channel can be modified so as to overexpress that protein relative to expression of the full-length protein (i.e. control assay). Typically, the modified protein will exhibit at least 10 percent or greater overexpression relative to the full-length protein; more preferably at least 20 percent or greater; and still more preferably at least about 30, 40, 50, 60, 70, 80, 100, 150, or 200 percent or greater overexpression relative to the control assay.

As noted above, further contemplated modifications to a polynucleotide (nucleic acid segment) and particularly a cDNA are those which create dominant negative proteins.

In general, a variety of dominant negative proteins can be made by methods known in the field. For example, ion channel proteins are recognized as one protein family for which dominant negative proteins can be readily made, e.g., by removing selected transmembrane domains. In most cases, the function of the ion channel binding complex is substantially reduced or eliminated by interaction of a dominant negative ion channel protein.

Several specific strategies have been developed to make dominant negative proteins. Exemplary of such strategies include oligonucleotide directed and targeted deletion of cDNA sequence encoding the desired protein.

It is stressed that creation of a dominant negative protein is not synonymous with other conventional methods of gene manipulation such as gene deletion and antisense RNA. What is meant by "dominant negative" is specifically what is sometimes referred to as a "poison pill" which can be driven (i.e. expressed) by an appropriate DNA construct to produce a dominant negative protein which has capacity to inactivate an endogenous protein.

For example, in one approach, a cDNA encoding a protein comprising one or more transmembrane domains is modified so that at least 1 and preferably 2, 3, 4, 5, 6 or more of the transmembrane domains are eliminated. Preferably, the resulting modified protein forms a binding complex with at least one other protein and usually more than one other protein. As noted, the modified protein will inhibit normal function of the binding complex as assayed, e.g., by standard ligand binding assays or electrophysiological assays as described herein. Exemplary binding complexes are those which participate in electrical charge propagation such as those occurring in ion channel protein complexes. Typically, a dominant negative protein will exhibit at least 10 percent or greater inhibition of the activity of the binding complex; more preferably at least 20 percent or greater; and still more preferably at least about 30, 40, 50, 60, 70, 80, or 100 percent or greater inhibition of the binding complex activity relative to the full-length protein.

As a further illustration, a cDNA encoding a desired protein for use in the present methods can be modified so that at least one amino acid of the protein is deleted. The deleted amino acid(s) can be contiguous or non-contiguous deletions essentially up to about 1%, more preferably about 5%, and even more preferably about 10, 20, 30, 40, 50, 60, 70, 80, or 95% of the length of the full-length protein sequence.

Alternatively, the cDNA encoding the desired protein can be modified so that at least one amino acid in the encoded protein is substituted by a conservative or non-conservative amino acid. For example, a tyrosine amino acid substituted with a phenylalanine would be an example of a conservative amino acid substitution, whereas an arginine replaced with an alanine would represent a non-conservative amino acid substitution. The substituted amino acids can be contiguous or non-contiguous substitutions essentially up to about 1%, more preferably about 5%, and even more preferably about 10, 20, 30, 40, 50, 60, 70, 80, or 95% of the length of the full-length protein sequence.

Although generally less-preferred, the nucleic acid segment encoding the desired protein can be modified so that at least one amino acid is added to the encoded protein. Preferably, an amino acid addition does not change the ORF of the cds. Typically, about 1 to 50 amino acids will be added to the encoded protein, preferably about 1 to 25 amino acids, and more preferably about 2 to 10 amino acids. Particularly preferred addition sites are at the C- or N-terminus of the selected protein.

Preferred invention practice involves administering at least one of the foregoing polynucleotides with a suitable myocardium nucleic acid delivery system. In one embodiment, that system includes a non-viral vector operably linked to the polynucleotide. Examples of such non-viral vectors include the polynucleoside alone or in combination with a suitable protein, polysaccharide or lipid formulation.

As used herein, the term "operably linked" refers to an arrangement of elements wherein the components so described are configured so as to perform their usual function. Thus, control sequences operably linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

As used herein, the term "coding sequence" or a sequence which "encodes" a particular protein, is a nucleic acid sequence which is transcribed (in the case of DNA) and translated (in the case of mRNA) into a polypeptide in vitro or in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from prokaryotic or eukaryotic mRNA, genomic DNA sequences from prokaryotic or eukaryotic DNA, and even synthetic DNA sequences. A transcription termination sequence will usually be located 3' to the coding sequence.

As used herein, "nucleic acid" sequence refers to a DNA or RNA sequence. The term also captures sequences that include any of the known base analogues of DNA and RNA such as, but not limited to, 4-acetylcylosine, 8-hydroxy-N6-methyladenosine, aziridinylcytosine, pseudoisocytosine, 5-(carboxyhydroxylmethyl)uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudo-uracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, 2-thiocytosine, and 2,6-diaminopurine.

As used herein, the term "control sequences" refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell.

As used herein, "promoter region" is used herein in its ordinary sense to refer to a DNA regulatory sequence to which RNA polymerase binds, initiating transcription of a downstream (3' direction) coding sequence.

A particularly preferred myocardium nucleic acid system, especially when it is desirable to suppress a certain activity such as $I_{K1}$ is described in US Patent No. 6,214,620 to D.C. Johns and E. Marban, the contents of which are hereby incorporated by reference in their entirety. Such a construct is controlled by use of an inducible promoter. Examples of such promoters, include, but not limited to those regulated by hormones and hormone analogs such as progesterone, ecdysone and glucocorticoids as well as promoters which are regulated by tetracycline, heat shock, heavy metal ions, interferon, and lactose operon activating compounds. For review of these systems see Gingrich and Roder, 1998, Ann. Rev. Neurosci., 21, 377-405. When using non-mammalian induction systems, both an inducible promoter and a gene encoding the receptor protein for the inducing ligand are employed. The receptor protein typically binds to the inducing ligand and then directly or indirectly activates transcription at the inducible promoter.

Additional suitable myocardium nucleic acid delivery systems include viral vector, typically sequence from at least one of an adenovirus, adenovirus-associated virus (AAV), helper-dependent adenovirus, retrovirus, or hermagglutinating virus of Japan-liposome (HVJ) complex. Preferably, the viral vector comprises a strong eukaryotic promoter operably linked to the polynucleotide eg., a cytomegalovirus (CMV) promoter.

Additionally preferred vectors include viral vectors, fusion proteins and chemical conjugates. Retroviral vectors include moloney murine leukemia viruses and HIV-based viruses. One preferred HIV-based viral vector comprises at least two vectors wherein the *gag* and *pol* genes are from an HIV genome and the *env* gene is from another virus. DNA viral vectors are preferred. These vectors include pox vectors such as orthopox or avipox vectors, herpesvirus vectors such as a herpes simplex I virus (HSV) vector [Geller, A.I. et al., J. Neurochem, 64:487 (1995); Lim, F., et al., in DNA Cloning: Mammalian Systems, D. Glover, Ed. (Oxford Univ. Press, Oxford England) (1995); Geller, A.I. et al., Proc Natl. Acad. Sci.: U.S.A.:90 7603 (1993); Geller, A.I., et al., Proc Natl. Acad. Sci USA: 87:1149 (1990)], Adenovirus Vectors [LeGal LaSalle et al., Science, 259:988 (1993); Davidson, et al., Not. Genet 3: 219 (1993); Yang, et al., J. Virol. 69: 2004 (1995)] and Adeno-associated Virus Vectors [Kaplitt, M.G., et al., Nat. Genet. 8:148 (1994)].

Pox viral vectors introduce the gene into the cells cytoplasm. Avipox virus vectors result in only a short term expression of the nucleic acid. Adenovirus vectors, adeno-associated virus vectors and herpes simplex virus (HSV) vectors are may be indication for some invention embodiments. The adenovirus vector results in a shorter term expression (e.g., less than about a month) than adeno-associated virus, in some embodiments, may exhibit much longer expression.

The particular vector chosen will depend upon the target cell and the condition being treated. Preferred *in vivo* or *ex vivo* cardiac administration techniques have already been described.

To simplify the manipulation and handling of the polynucleotides described herein, the nucleic acid is preferably inserted into a cassette where it is operably linked to a promoter. The promoter must be capable of driving expression of the protein in cells of the desired target tissue. The selection of appropriate promoters can readily be accomplished. Preferably, one would use a high expression promoter. An example of a suitable promoter is the 763-base-pair cytomegalovirus (CMV) promoter. The Rous sarcoma virus (RSV) (Davis, et al., Hum Gene Ther 4:151 (1993)) and MMT promoters may also be used. Certain proteins can be expressed using their native promoter. Other elements that can enhance expression can also be included such as an enhancer or a system that results in high levels of expression such as a *tat* gene and *tar* element. This cassette can then be inserted into a vector, e.g., a plasmid vector such as pUC118, pBR322, or other known plasmid vectors, that includes, for example, an *E. coli* origin of replication. See, Sambrook, et al., Molecular Cloning: A Laboratory Manual, Cold Spring Harbor Laboratory press, (1989). The plasmid vector may also include a selectable marker such as the β-lactamase gene for ampicillin resistance, provided that the marker polypeptide does not adversely effect the metabolism of the organism being treated. The cassette can also be bound to a nucleic acid binding moiety in a synthetic delivery system, such as the system disclosed in WO 95/22618.

U.S. Published Patent Application US20020022259A1 also reports polynucleotide enhancer elements for facilitating gene expression in cardiac cells and differentiating stem cells to cardiomyocytes.

If desired, the polynucleotides of the invention may also be used with a microdelivery vehicle such as cationic liposomes and adenoviral vectors. For a review of the procedures for liposome preparation, targeting and delivery of contents, see Mannino and Gould-Fogerite, BioTechniques, 6:682 (1988). See also, Felgner and Holm, Bethesda Res. Lab. Focus, 11(2):21 (1989) and Maurer, R.A., Bethesda Res. Lab. Focus, 11(2):25 (1989).

Replication-defective recombinant adenoviral vectors, can be produced in accordance with known techniques. See, Quantin, et al., Proc. Natl. Acad. Sci. USA, 89:2581-2584 (1992); Stratford-Perricadet, et al., J. Clin. Invest., 90: 626-630 (1992); and Rosenfeld, et al., Cell, 68:143-155 (1992).

One preferred myocardicum delivery system is a recombinant viral vector that incorporates one or more of the polynucleotides therein, preferably about one polynucleotide. Preferably, the viral vector used in the invention methods has a pfu (plague forming units) of from about $10^8$ to about 5x $10^{10}$ pfu. In embodiments in which the polynucleotide is to be administered with a non-viral vector, use of between from about 0.1nanograms to about 4000 micrograms will often be useful e.g., about 1 nanogram to about 100 micrograms.

Choice of a particular myocardium delivery system will be guided by recognized parameters including the condition being treated and the amount and length of expression desired. Use of virus vectors approved for human applications e.g., adenovirus are particularly preferred.

Reference herein to an electrophysiological assay is meant a conventional test for determining cardiac action potential (AP). See generally Fogoros RN. Electrophysiologic Testing Blackwell Science, Inc. (1999.) for disclosure relating to performing such tests.

Specific reference herein to a "standard electrophysiological assay" is meant the following general assay.

1) providing a mammalian heart (*in vivo* or *ex vivo*),
2) contacting the heart with at least one suitable polynucleotide preferably in combination with an appropriate myocardium nucleic acid delivery system, or with modified cells as disclosed herein such as stem cells that have differentiated to cardiomyocytes,
3) transferring the polynucleotide or modified cells into the heart and under conditions which can allow expression of the encoded amino acid sequence; and
4) detecting modulation (increase or decrease) of at least one electrical property in the administered (e.g. transformed) heart e.g., at least one of conduction, ventricular response rate, firing rate and/or pulse rate, preferably firing rate or pulse rate, relative to a baseline value. As will be appreciated, baseline values will often vary with respect to the particular polynucleotide(s) chosen. Methods to quantify baseline expression or protein include western blot, quantitative PCR, or functional assays such as adenylate cyclase assay for inhibitory G proteins, patch clamp analysis for ion channel currents. Electrophysiology (EP) effects can be determined by measuring heart rate, conduction velocity or refractory period *in vivo* with EP catheters. Preferred rates of modulation are at least 1, 2, 3, 4, 5, 6, 7, 8, 9, or 10 percent difference from a baseline value. Greater increases or decreases from a baseline value also may be achieved e.g. an increase or decrease of heart rate or other measured property of at least about 12, 15, 20 or 25 percent relative to a baseline value.

Particular invention methods include modifying the polynucleotide along lines discussed above sufficient to overexpress the encoded protein. Further preferred are methods in which the nucleic acid is modified to produce a dominant negative ion channel protein. The ion channel protein can be a voltage-gated (such as sodium, calcium, or potassium channel) or a ligand-gated ion channel. Additional disclosure relating to such channel proteins can be found in the discussion above and in U.S. Pat. No. 5,436,128, for instance.

Practice of the invention is broadly compatible with one or a combination of different administration (delivery) systems.

In particular, one suitable administration route involves one or more appropriate polynucleotide into myocardium. Alternatively, on in addition, the administration step includes perfusing the polynucleotide into cardiac vasculature. If desired, the administration step can further include increasing microvascular permeability using routine procedures, typically administering at least one vascular permeability agent prior to or during administration of the gene transfer vector. Examples of particular vascular permeability agents include administration of one or more of the following agents preferably in combination with a solution having less than about 500 micromolar calcium: substance P, histamine, acetylcholine, an adenosine nucleotide, arachidonic acid, bradykinin, endothelin, endotoxin, interleukin-2, nitroglycerin, nitric oxide, nitroprusside, a leukotriene, an oxygen radical, phospholipase, platelet activating factor, protamine, serotonin, tumor necrosis factor, vascular endothelial growth factor, a venom, a vasoactive amine, or a nitric oxide synthase inhibitor. A particular is serotonin, vascular endothelial growth factor (VEGF), or a functional VEGF fragment to increase the permeability.

Typical perfusion protocols in accord with the invention are generally sufficient to transfer the polynucleotide to at least about 10% of cardiac myocytes in the mammal. Infusion volumes of between from about 0.5 to about 500 ml are preferred. Also preferred are coronary flow rates of between from about 0.5 to about 500 ml/min. Additionally preferred perfusion protocols involve the AV nodal artery. Transformed heart cells, typically cardiac myocytes that include the polynucleotide are suitably positioned at or near the AV node.

Illustrative strategies for detecting modulation of transformed heart have been disclosed e.g., in Fogoros RN, supra. A preferred detection strategy is performing a conventional electrocardiogram (ECG). Modulation of cardiac electrical properties by use of the invention is readily observed by inspection of the ECG.

More generally, the invention can be used to deliver and express a desired ion channel, extracellular receptor, or intracellular signaling protein gene in selected cardiac tissues, particularly to modify the electrical properties of that tissue, e.g., increasing or decreasing the heart rate, increasing or decreasing its refractoriness, increasing or decreasing the speed of conduction, increasing or decreasing focal automaticity, and/or altering the spatial pattern of excitation. The general method involves delivery of genetic materials (DNA, RNA) by injection of the myocardium or perfusion through the vasculature (arteries, veins) or delivery by nearly any other material sufficient to facilitate transformation into the targeted portion of the myocardium using viral (adenovirus, AAV, retrovirus, HVJ, other recombinant viruses) or non-viral vectors (plasmid, liposomes, protein-DNA combinations, lipid-DNA or lipid-virus combinations, other non-viral vectors) to treat cardiac arrhythmias.

By way of illustration, genes that could be used to affect cardiac firing rate include ion channels and pumps ($\alpha$ subunits or accessory subunits of the following: potassium channels, sodium channels, calcium channels, chloride channels, stretch-activated cation channels, HCN channels, sodium-calcium exchanger, sodium-hydrogen exchanger, sodium-potassium ATPase, sarcoplasmic reticular calcium ATPase), cellular receptors and intracellular signaling pathways ($\alpha$ or $\beta$-adrenergic receptors, cholinergic receptors, adenosine receptors, inhibitory G protein $\alpha$ subunits, stimulatory G protein $\alpha$ subunits, G$\beta\gamma$ subunits) or genes for proteins that affect the expression, processing or function processing of these proteins.

As discussed above, modified cells also may be administered to induce or modulate pacemaker activity of cells or a subject. Once source of modified cells are cardiac myocardial cells generated from differentiated (spontaneous or driven) stem cells, such as embryonic bone marrow cells. The stem-cell-derived cardiomyocytes exhibiting pacemaker function then may be implanted such as by catheter or injection to targeted cardiac tissue. Methods suitable for producing stem cell-derived cardiac myocytes are disclosed in e.g. U.S. Published Patent Application US20010024924A1 and U.S. Published Patent Application US20020022259A1.

Similarly, existing cardiomyocytes may be transformed with a polynucleotide expression to provide desired pacemaker as discussed herein *ex vivo* and then implanted to targeted cardiac tissue of a subject e.g. by catheter or injection. Suitably, the existing cardiomyocytes may be harvested from the subject receiving treatment to facilitate delivery of those cells after modification (e.g. transformed with a polynucleotide expression system as disclosed herein) and re-administration.

The modified cells may have been harvested from the recipient, i.e. the subject to which the cells are administered. For example, bone marrow stem cells may be harvested from a subject and then differentiated to cardiomyocytes with pacemaker function. Cardiac cells, such as sino-atrial node cells may be harvested from a subject such as through removal via catheter or other protocol, modified e.g. by insertion of a desired polynucleotide delivery system as disclosed herein and then administered to the subject. As discussed above, as referred to herein, the administered cells preferably are modified in some respect prior to administration, such as differentiated stem cells or transformed with a polynucleotide expression system; modified administered cells as referred to herein would not include simply transplanted cardiac cells that had not been modified in some respect.

Preferred subjects for treatment in accordance with the invention include domesticated animals e.g., pigs, horses, dogs, cats, sheep, goats and the like; rodents such as rats, hamsters and mice; rabbits; and primates such as monkeys, chimpanzees etc. A highly preferred mammal is a human patient, preferably a patient who has need of or suspected of having need of cardiac rhythm disorder, such as those disclosed herein.

Preferred subject for treatment include those that are suffering from or susceptible to a disease or disorder as disclosed herein e.g. such as a cardiac-related syncope, particularly Stokes-Adam syncope; an abnormality of sinus node function such as persistent sinus bradycardia, sino-atrial (S-A) block manifested as S-A Wenckebach, complete S-A block or sinus arrest, and high-grade atriventricular block; or bradycardia-tachycardia syndrome or other bradycardia related condition.

The effective dose of the nucleic acid will be a function of the particular expressed protein, the particular cardiac arrhythmia to be targeted, the patient and his or her clinical condition, weight, age, sex, etc.

More specific advantages of the invention include ability to convey localized effects (by focal targeted gene delivery), reversible effects (by use of inducible vectors, including those already reported as well as new generations of such vectors, including but not limited to adeno-associated vectors using tetracycline-inducible promoters to express wild-type or mutant ion channel genes), gradedness (by use of inducible vectors as noted above, in which gradedness would be achieved by titration of the dosage of the inducing agent), specificity of therapy based on the identity of the gene construct, ability to regulate therapeutic action by endogenous mechanisms (nerves or hormones) based on the identity of the gene construct, and avoidance of implantable hardware including electronic pacemakers and AICDs, along with the associated expense and morbidity.

The following non-limiting examples are illustrative of the invention. All documents mentioned herein are incorporated herein by reference.

Example 1: *Effect of inhibition of Kir2 channels on latent pacemaker activity of ventricular myocytes.*

*Materials and Methods*

*Dominant-negative effects*

Dominant-negative effects of Kir2.1AAA on $I_{K1}$ expression were achieved using the approach of Herskowitz (See, for example I. Herskowitz, Nature 329, 219-22; (1987)). The GYG motif, three amino acids in the H5 region of potassium channels that play a key role in selectivity and pore function, were replaced with three alanines in Kir2.1.

*Vectors*

A bicistronic adenoviral vector, encoding both enhanced green fluorescence protein (EGFP, Clontech, Palo Alto, CA, USA) and Kir2.1AAA, was created using the adenovirus shuttle vectors pAdEGI (D. C. Johns, H. B. Nuss, E. Marban, J. Biol. Chem. 272, 31598-603. (1997) and pAdC-DBEcR (U. C. Hoppe, E. Marban, D. C. Johns, J. Clin. Invest. 105, 1077-84. (2000)) as previously described. The full-length coding sequence of human Kir2.1 (kindly supplied by G.F. Tomaselli, Johns Hopkins University) was cloned into the multiple cloning site of pAdEGI to generate pAdEGI-Kir2.1. The dominant-negative mutation GYG→AAA was introduced into Kir2.1 by site-directed mutagenesis, creating the vectorpAdEGI-Kir2.1AAA. Adenovirus vectors were generated by Cre-lox recombination of purified 5 viral DNA and shuttle vector DNA as described (D. C. Johns, R. Marx, R. E. Mains, B. O'Rourke, E. Marban, J Neurosci. 19, 1691-7. (1999)). The recombinant products were plaque purified, expanded, and purified on CsCl gradients yielding concentrations on the order of $10^{10}$ plaque-forming units (PFU) per milliliter.

*In vivo gene delivery*

Intracardiac injection was achieved by injection into the left ventricular cavity of adult guinea pigs (250-300 g) following lateral thoracotomy. The aorta and pulmonary artery were first cross-clamped and then a 30 gauge needle was inserted at the apex, enabling injection of the adenovirus solution into the left ventricular chamber. A total volume of 220 $\mu$l of adenovirus mixture was injected, containing 3 x $10^{10}$ PFU AdC-DBEcR and 2 x $10^{10}$ PFU AdEGI (control group) or 3 x $10^{10}$ PFU AdC-DBEcR and 3 x $10^{10}$ PFU AdEGI-Kir2.1AAA (knock-out group). The aorta and pulmonary artery remain occluded for 40-60 seconds before the clamp is released. This procedure allows the virus to circulate down the coronaries while the heart is pumping against a closed system and results in a widespread distribution of transduced cells. After the chest was closed, animals were injected intraperitoneally with 40 mg of the nonsteroidal ecdysone receptor agonist, GS-E ([N-(3-methoxy-2-ethylbenzoyl)-N'-(3,5-dimethylbenzoyl)-N'-tertbutylhydrazine]; kindly provided by Rohm and Haas Co., Spring House, Pennsylvania, USA), dissolved in 90 $\mu$l DMSO and 360 $\mu$l sesame oil.

*Transduction efficiency*

Transduction efficacy was assessed by histological evaluation of microscopic sections 48 hours after injection of AdCMV-βgal (160 μl of 2 x $10^3$ pfu/ml) into the LV cavity. After the animals were killed, hearts were excised, rinsed thoroughly in PBS, and cut into transverse sections. The sections were fixed in 2% formaldehyde/02% glutaraldehyde and stained in PBS containing 1.0 mg/ml 5-bromo-4-chloro-3-indolyl- -D-galactoside (X-gal) as previously described (J. K. Donahue et al,, Nat Med 6, 1395-8. (2000)). The sections were embedded in paraffin, cut to 5 μm thickness and stained with X-gal solution for visual assessment of transduction efficacy (J. K. Donahue et al., Nat. Med 6, 1395-8. (2000)). This gene delivery method achieved transduction of approximately 20% of ventricular myocytes throughout the LV wall.

Sixty to 72 hours after injection, guinea-pig left ventricular myocytes were isolated using Langendorff perfusion and collagenase digestion (R. Mitra, M. Morad, Proc Nail Acad Sci U SA 83, 5340-4. (1986); U. C. Hoppe, D. C. Johns, E. Marban, B. O'Rourke, Circ Res 84, 964-72. (1999). Dissociations typically yielded 60-70% viable myocytes. A xenon arc lamp was used to view GFP fluorescence at 488/530 nm (excitation/emission). Transduced myocytes were identified by their green fluorescence using epifluorescence. The yield of transduced and viable isolated myocytes using the LV cavity injection approach (-20%) was much higher than with direct intramyocardial injection (U. C. Hoppe, E. Marban, D. C. Johns, J Clin Invest 105, 1077-84. (2000); U. C. Hoppe, E. Marban, D. C. Johns, Proc Natl Acad Sci U S A 98, 5335-40. (2001)).

Cellular recordings were performed using the vvhole-cell patch clamp technique (*24*) with an Axopatch 200B amplifier (Axon Instruments, Foster City, California, USA) while sampling at 10 kHz (for currents) or 2 kHz (for voltage recordings) and filtering at 2 kHz. Pipettes had tip resistances of 2-4 MΩ when filled with the internal recording solution. Cells were superfused with a physiological saline solution containing (in mM) 140 NaCl, 5 KCl, 2 $CaCl_2$, 10 glucose, 1 $MgCl_2$, 10 HEPES; pH was adjusted to 7.4 with NaOH. For $I_{K1}$ recordings, $CaCl_2$ was reduced to 100 pM, $CdCl_2$ (200 μM) was added to block $I_{caL}$, and $I_{Na}$ was steady-state inactivated by using a holding potential of -40 mV. To obtain $I_{K1}$ as a barium ($Ba^{2+}$)-sensitive current, background currents remaining after the addition of $Ba^{2+}$ (500 μM) were subtracted from the records. The pipette solution was composed of (in mM) 130 K-glutamate, 19 KCl, 10 Na-Hepes, 2 EGTA, 5 Mg-ATP, 1 $MgCl_2$; pH was adjusted to 7.2 with KOH. Data were not corrected for the measured liquid junction potential of -12 mV. Action potentials were initiated by brief depolarizing current pulses (2 ms, 500-800 pA, 110% threshold) at 0.33 Hz. Action potential duration (APD) was measured as the time from the overshoot to 50% or 90% repolarization ($APD_{50}$, $APD_{90}$, respectively). For $I_{Ca,L}$ recordings, cells were superfused with a saline solution containing (in mM) 140 N-methyl-D-glucanine, 5 CsCl, 2 $CaCl_2$, 10 glucose, 0.5 $MgCl_2$, 10 HEPES; pH was adjusted to 7.4 with HCl The pipette solution was composed of (in mM) 125 CsCl, 20 TEA-Cl, 2 EGTA, 4 Mg-ATP, 10 HEPES; pH was adjusted to 7.3 with CsOH. Data reported are means $\pm$ S.E.M. with P<0.05 (t test) indicating statistical significance.

All recordings were performed at physiologic temperature (37 °C) and 60-72 hours after in vivo transduction. Given that adenovirus infection itself does not modify the electrophysiology of guinea-pig myocytes (U. C. Hoppe, E. Marban, D. C. Johns, J Clin Invest 105, 1077-84. (2000)), patch-clamp experiments performed on nontransduced (non-green) left ventricular myocytes isolated from AdEGI-Kir2.1AAA-injected animals ($APD_{50}$= 233.8$\pm$10.5 ms, n=6), as well as on green cells from AdEGI-injected hearts ($APD_{50}$= 247.6$\pm$10.3 ms, n=24, P=0.52), were used as controls.

*Electro cardiographs*

Surface ECGs were recorded immediately after operation and 72 hours after intramyocardial injection as previously described (U. C. Hoppe, E. Marban, D. C. Johns, J Clin Invest 105, 1077-84. (2000); U. C. Hoppe, E. Marban, D. C. Johns, Proc Natl Accrd Sci U S A 98, 5335-40. (2001)). Guinea pigs were sedated with isoflurane, and needle electrodes were placed under the skin. Electrode positions were optimized to obtain maximal amplitude recordings, enabling accurate measurements of QT intervals. ECGs were simultaneously recorded from standard lead 11, and modified leads I and III. The positions of the needle electrodes were marked on the guinea pigs' skin after recording, to ensure exactly the same localization 72 hours later. The rate corrected-QT interval (QTc) was calculated (E. Hayes, M. K. Pugsley, W. P. Penz, G. Adaikan, M. J. Walker, J Pharmcacol. Toxicol. Methods 32, 201-7. (1994)).

*Results*

Replacement of three critical residues in the pore region of Kir2.1 by alanines ($GYG_{144-146} \rightarrow$ AAA, or Kir2.1AAA) creates a dominant-negative construct which suppresses current flux when co-expressed with wild-type Kir2.1. In oocytes, injection of Kir2.1AAA RNA alone does not produce current, but coinjection with wild-type Kir2.1 RNA causes suppression of $I_{Kl}$. Incorporation of a single mutant subunit within the tetrameric Kir channel is sufficient to knock out function. The dominant-negative effects are specific to the Kir family of potassium channels, as Kv1.2 currents were not reduced by co-injection with Kir2.1AAA RNA. When Kir2.1AAA was transiently expressed in a mammalian cell line (HEK) stably expressing Kir2.1, the dominant-negative construct reduced $I_{K1}$ by approximately 70%.

Kir2.1AAA was packaged into a bicistronic adenoviral vector and injected into the left ventricular cavity of guinea pigs. This method of delivery sufficed to achieve transduction of ~20% of ventricular myocytes (Figure 1). Myocytes isolated 3-4 days after *in vivo* transduction with Kir2.1AAA exhibited suppression of $I_{K1}$ (Figure 2 B,C), but calcium currents remained unchanged (Figure 2 E,F).

Control ventricular myocytes exhibited no spontaneous activity, but did fire single action potentials when subjected to depolarizing external stimuli (Figure 3A). In contrast, Kir2.1AAA myocytes exhibited either of two phenotypes: a stable resting potential from which prolonged action potentials could be elicited by external stimuli (Figure 3C, "long QT phenotype"), or spontaneous activity (Figure 3E). Prolongation of action potentials would be expected to lengthen the QT interval of the electrocardiogram (long QT phenotype), whereas the spontaneous activity resembles that of genuine pacemaker cells; the maximum diastolic potential is relatively depolarized, with repetitive, regular and incessant electrical activity initiated by gradual "phase 4" depolarization and a slow upstroke (Table 1). The different phenotypes correspond to three distinct ranges of $I_{K1}$ density (Figure 2 B,D,F,G). Thus, Kir2.1AAA-transduced myocytes exhibit either a long QT phenotype or a pacemaker phenotype, depending upon how much suppression of $I_{K1}$ happened to have been achieved in that particular cell. Myocytes in which $I_{K1}$ was suppressed below 0.4 pA/pF (at -50 mV) all exhibited spontaneous AP, while myocytes with greater than 0.4 pA/pF $I_{K1}$ had stable resting membrane potentials and prolonged action potentials.

Cells with a pacemaker phenotype were unaffected by the Na channel blocker tetrodotoxin (Figure 4 A,B), but spontaneous firing ceased during exposure to calcium channel blockers (cadmium, Figure 4 C,D; nifedipine, E,F). Thus, the excitatory current underlying spontaneous action potentials is carried by calcium channels, as is the case with genuine pacemaker cells. Likewise, Kir2.1AAA spontaneous-phenotype cells responded to beta-adrenergic stimulation just as nodal cells do, increasing their pacing rate (Figure 5) to accelerate the heart rate.

Table 2. Action potential characteristics in control, long QT phenotype Kir2.1AAA, and pacemaker phenotype Kir2.1AAA myocytes. In all of the control (n=30) and long QT phenotype Kir2.1AAA cells (7 of 22 Kir2.1AAA cells), stable action potentials were evoked in response to electrical stimulation. The pacemaker phenotype Kir2.1AAA cells (15 of 22 Kir2.1AAA cells) exhibited the spontaneous action potentials with no input stimulus.

| Cells | Maximum diastolic potential (mV) | Spontaneous action potential rate (APs/min) | Maximum upstroke velocity (V/s) | APD$_{50}$ (ms) | APD$_{90}$ (ms) |
|---|---|---|---|---|---|
| Control | -75.3 $\pm$ 0.7 | N/A | 101.3 $\pm$ 3.3 | 244.8 $\pm$ 85 | 271.1 $\pm$ 85 |
| Long QT phenotype Kir2.1AAA | -68.0 $\pm$ 2-3* | N/A | 92.4 $\pm$ 7.0 | 271.9 $\pm$ 19.5 | 353.4 $\pm$ 17.4* |
| Pacemaker phenotype Kir2.1AAA | -60.7 $\pm$ 2.1* | 116.8 $\pm$ 10.9* | 15.2 $\pm$ 4.5* | 173.7 $\pm$ 13.2* | 232.8 $\pm$ 20.3* |

* $P<0.05$ Kir2:1AAA vs. control

APD$_{50}$ and APD$_{90}$ are measurements of action potential duration taken from the AP overshoot to 50% or 90% repolarization (APD$_{50}$, APD$_{90}$, respectively).

Electrocardiography revealed two phenotypes. Figure 6A shows a prolongation of the QT interval (Figure 6A). Nevertheless, 40% of the animals exhibited an altered cardiac rhythm indicative of spontaneous ventricular foci (Figure 6B). Premature beats of ventricular origin can be distinguished by their broad amplitude, and can be seen to "march through" to a beat independent of that of the physiological sinus pacemaker. In normal sinus rhythm, every P wave is succeeded by a QRS complex. However, if ectopic beats arise from foci of induced pacemakers, the entire heart can be paced from the ventricle. Indeed, ventricular automaticity developed in two of five animals 72 hours after transduction with Kir2.1AAA. In these two animals, P waves were not followed by QRS complexes; both P waves and QRS complexes maintained independent rhythms. The RR intervals were shorter than the PP intervals, signifying a rhythm of ventricular origin (accelerated ventricular rhythm due to automaticity). The two phenotypes *in vivo* correspond well to the distinct long QT and pacemaker cellular phenotypes.

The dominant negative results demonstrate the durability and regional specificity of the methods used herein. These results demonstrate that the specific suppression of Kir2 channels suffices to unleash pacemaker activity in ventricular myocytes. These results also demonstrate that the important factor for pacing is solely the absence of the strongly-polarizing $I_{K1}$, rather than the presence of special genes (although such genes may play an important modulatory role in genuine pacemaker cells).

Example 2: *The triple mutation GYG$_{365-367}$AAA rendered HCN1 channels non functional.*

*Molecular Biology and Heterologous Expression*

mHCN1 and mHCN2 were subcloned into the pGH expression vector. B. Santoro et al., Cell, 93:717-29 (1998). Site-directed mutagenesis was performed using polymerase chain reaction (PCR) with overlapping mutagenic primers. All constructs were sequenced to ensure that the desired mutations were present. cRNA was transcribed from Nhel- and Sphl-linearized DNA using T7 RNA polymerase (Promega, Madison, WI) for HCN1 and HCN2 channels, respectively. Channel constructs were heterologously expressed and studied in *Xenopus* oocytes. Briefly, stage IV through VI oocytes were surgically removed from female frogs anesthetized by immersion in 1% tricaine (i.e. 3-aminobenzoic acid ethyl ester) followed by digestion with 2 mg/mL collagenase in OR-2 containing (in mM): 88 NaCl, 2 KCl, 1 MgCl2 and 5 mM HEPES (pH 7.6 with NaOH) for 30 to 60 minutes. Isolated oocytes were injected with cRNA (Ing/nL) as indicated, and stored in ND96 solution containing (in mM) 96 NaCl, 2 KCl, 1.8 CaCl$_2$, 1 MgCl$_2$ and 5 HEPES (pH 7.6) supplemented with 50 $\mu$g/mL gentamicin, 5 mM pyruvate and 0.5 mM theophylline for 1-4 days before experiments. It was found that injection with 50-100 ng of total cRNA per cell was sufficient to attain maximal expression while 10-25 ng/cell corresponds to the range linearly proportional to the expressed current amplitude.

*EZectrophysiology*

Two-electrode voltage-clamp recordings were performed at room temperature (23-25°C) using a Warner OC-725C amplifier (Hamden, CT). Agarose-plugged electrodes (TW120F-6; World Precision Instruments) were pulled using a Sutter P-87 horizontal puller, filled with 3 M KCl and had final tip resistances of 2-4 Ma The recording bath solution contained (in mM): 96 KCl, 2 NaCl, 10 HEPES, and 2 MgCl$_2$ (pH 7.5 with KOH). The currents were digitized at 10 kHz and low-pass filtered at 1-2 kHz (-3 dB). Acquisition and analysis of current records were performed using custom-written softwares.

*Experimental Protocols and Data analysis*

The steady-state current-voltage (I-V) relationship was determined by plotting the HCN1 currents measured at the end of a 3-second pulse ranging from - 150 to 0 mV at 10 mV increments from a holding potential of -30 mV. The voltage dependence of HCN channel activation was assessed by plotting tail currents measured immediately after pulsing to -140 mV as a function of the preceding 3-second test pulse normalized to the maximum tail current recorded. Data were fit to the Boltzmann functions using the Marquardt-Levenberg algorithm in a non-linear least-squares procedure:

$$m \; = 1 \; / \; \{1 + \exp[(V_t - V_{1/2}) / k]\}$$

where $V_t$ is the test potential, $V_{1/2}$ is the half-point of the relationship and k =RT/zF is the slope factor.
For reversal potentials ($E_{rev}$), tail currents were recorded immediately after stepping to a family of test voltages ranging from -100 to +40 mV preceded by a 3-s prepulse to either -140 (cf. Figure 11B) or -20 mV. The difference of tail currents resulting from the two prepulse potentials was plotted against the test potentials, and fitted with linear regression to obtain $E_{rev}$. For current kinetics, the time constants for activation ($\tau_{act}$) and deactivation ($\tau_{deact}$) were estimated by fitting macroscopic and tail currents, respectively, with a mono-exponential function.
Data are presented as mean $\pm$ SEM. Statistical significance was determined using an unpaired Student's t-test with p<0.05 representing significance.
The effects of the triple HCN1 mutation GYG$_{365-367}$AAA (HCN1-AAA) on channel function were evaluated by individually expressing WT and mutant channel constructs. Figure 8 shows that hyperpolarization of oocytes injected with WT HCN1 cRNA to potentials below -40 mV elicited time-dependent inward currents that reached steady state current amplitudes after ~500 ms. Currents increased in amplitude with progressive hyperpolarization. In contrast, uninjected oocytes and those injected with HCN1-AAA did not yield measurable currents, indicating that the triple alanine substitution rendered HCN1 completely non-functional.

*HCN1 AAA suppressed the normal activity of WT HCN1 in a dominant-negative manner*

Previous studies have identified numerous ion channel mutations that are capable of crippling channel activities in a dominant-negative manner when normal and defective subunits coassemble to form multimeric complexes. R. Li et al., J. Physiol, 533: 127-33 (2001); J. Seharaseyon, J. Mol. Cell Cardiol., 32:1923-30 (2000); MT Perez-Carcia, J. Neurosci, 20:5689-95 (2000); J. Seharseyon et al., J. Biol Chem., 275: 17561-5 (2000); UC Hoppe et al., J. Clin Invest, 105:1077-84 (2000); MJ Lalli et al., Pflugers Arch., 436:957-61 (1998); DC John et al., J. Biol Chem, 272:31598-603 (1997). HCN1-AAA was anticipated to exert a dominant-negative effect when combined with WT HCN1 subunits. That was tested by co-expressing both WT HCN1 and HCN1-AAA channel constructs. Figure 9 shows that oocytes co-injected with 50nL WT HCN1 and 50nL HCN1-AAA cRNA (concentration=1ng/nL) expressed currents $85.2 \pm 1.9\%$ (n=8) smaller than cells injected with 50nL of WT HCN1 cRNA alone when measured at - 140 mV after the same incubation period (p<0.01; Figure 9B). Such quantitative differences existed throughout almost the entire activation range of HCN1 channels as indicated by their corresponding steady-state current-voltage relationships (Figure 9C). These observations demonstrate that HCN1-AAA could suppress the normal activity of WT HCN1 channels in a dominant-negative fashion despite the presence of the same numbers of functional subunits (assuming equal RNA stability and translation efficiencies, as is conventional in previous $K^+$ channel studies, Hille B. Ion channels of Excitable Membranes. 3rd Edition. Sunderland, Massachusetts, U.S.A. Sinauer Associates, Inc. 2001; R. MacKinnon Nature. 1991;350:232-5.). In contrast, co-injection of 50nL WT HCN1 cRNA with an equal volume of $dH_2 0$ yielded current magnitudes not different from the injection of 50 nL WT HCN1 alone (p>0.05), suggesting that the dominant-negative suppressive effects observed with HCN1-AAA were not due to non-specific mechanisms such as mechanosensitive effects. We also studied the effects of varying the ratio of WT HCN 1:HCN 1-AAA and WT HCN2:HCN 1-AAA while maintaining the total cRNA injected constant (25 ng was used to prevent saturation of expression). As anticipated from a dominant-negative mechanism, current suppression increased as the proportion of HCN1-AAA increased ($55.2 \pm 3.2$, $68.3 \pm 4.3$, $78.7 \pm 1.6$, $91.7 \pm 0.8$, $97.9 \pm 0.2\%$ current reduction for WT HCN1:HCN1-AAA ratios of 4:1, 3:1, 2:1, 1:1 and 1:2, respectively; Figure 10).

*Co-expression of the dominant-negative construct HCN1-AAA with WT HCN1 did not alter normal gating and permeation properties*

The affect of co-expression of HCN1-AAA with WT HCN1 on gating and permeation properties in addition to its dominant-negative suppressive effects on current amplitudes were then investigated. Figure 11A shows that both the midpoints and slope factors derived from the steady-state activation curves of WT HCN1 alone ($V_{1/2}$= -76.7 $\pm$ 0.8 mV; k= 13.3 $\pm$ 0.6 mV; n=15) and after suppression by HCN1-AAA (ratio=1:1; $V_{1/2}$= -77.0 $\pm$ 1.7 mV; k= 12.3 $\pm$ 1.0 mV; n=12) were identical (p>0.05). Tail current-voltage relationships also indicate that whereas whole-cell currents were suppressed by HCN1-AAA, the reversal potential was not changed (WT HCN1 alone = -4.5 $\pm$ 1.4 mV, n=8; WT+AAA = - 5.25 $\pm$ 0.8 mV, n=5; p>0.05; Figure11B&C). Similarly, the time constants for current activation ($\tau_{deact}$) and deactivation ($\tau_{deact}$), whose distribution was bell-shaped with midpoints comparable to those derived from the corresponding steady-state activation curves, were also unaltered after HCN1-AAA suppression across the entire voltage range studied (p>0.05; Figure 11D). Taken together, our observations indicate that the non-suppressed currents exhibited normal gating and permeation phenotypes.

*HCN1 AAA suppressed WT HCN2 currents without altering gating and permeation.*

If different HCN isoforms can coassemble to form heteromeric channel complexes, HCN1-AAA should also suppress the activities of WT HCN2 channels in a dominant-negative manner similar to our observations with WT HCN1. Figure 12 shows that this was indeed the case. Currents recorded from oocytes co-injected with 50nL WT HCN2 and 50nL HCN1-AAA cRNA were significantly smaller than those expressed in oocytes injected with 50nL WT HCN2 alone or 50nL WT HCN2+50nL dH20 after the same incubation period (Figure 12A-C). In fact, the extents of suppression by HCN1-AAA were similar for both WT HCN1 and HCN2 for all other ratios studied (Figure 10; total cRNA injected=25 ng). Taken together, these results indicate that the two isoforms were able to coassemble with equivalent efficacy. Similar to HCN1, steady-state activation parameters, reversal potential, and gating kinetics of the non-suppressed HCN2 currents were not changed by HCN1-AAA coexpression (p>0.05; Figure 12D-F).

*Engineered HCN1 channels exhibit channel activation shifted in positive and negative directions.*

Modulatation of HCN channel gating properties by protein engineering also was accomplished. Figures 13A and B show that the charge-neutralizing substitutions E235A produced a significant depolarizing shift in steady-state channel activation ($V_{1/2}$=-59.2 $\pm$ 1.5 mV, n=7; p<0.05) with and insignificant change in the slope factor (k=12.3 $\pm$ 0.9 mV, n=7; p>0.05). Consistent with an electrostatic role of residue 235, the charge-reversed mutation E235R shifted the steady-state activation curve even more positively (56.4 $\pm$ 0.5 mV, n=3; p<0.05). Neither the slope factor (7.9 $\pm$ 0.8 mV, n=3; p>0.05) nor $P_{o,min}$ (17.0% $\pm$ 1.9%, n=3; p>0.05) was affected by E235R (p>0.05; Figures 13A and C). We next investigated whether the S4 serine variant at position 253 underlies the distinctive activation profile of HCN1 channels by multiple substitutions (Figure 8). Replacing S253 with alanine (i.e. S253A) produced parallel hyperpolarizing shifts in the steady-state activation relationship and the voltage-dependence of gating kinetics while slowing both activation and deactivation (Figure 13D). S253A, however, did not alter $P_{o, min}$. Despite the opposite charges of S253K and S253E, both substitutions shifted the steady-state I-V relationship in the same hyperpolarizing direction. Taken collectively, this shows that the activation threshold of HCN channel activity can be modulated (Figure 13) as well as the endogenous expressed current amplitude (Figures 8-12).

The invention has been described in detail with reference to preferred embodiments thereof. However, it will be appreciated that those skilled in the art, upon consideration of the disclosure, may make modification and improvements within the spirit and scope of the invention.

**Claims**

1. A polynucleotide encoding an amino acid sequence for a mutant hyperpolarization-activated, cyclic nucleotide gated (HCN) ion channel,
   wherein the amino acid sequence comprises three alanine molecules at positions 365 to 367 as compared to the sequence of a wild-type HCN1 channel, for the use in a method of modulating contraction function or electrical activity of myocardial cells,

   by administration to myocardial cells,
   wherein said myocardial cells are quiescent cells that have a first frequency of contraction or electrical activity, and
   whereby after administration the myocardial cells have a second frequency of contraction or electrical activity.

2. The polynucleotide for use according to claim 1, wherein expression of the polynucleotide after administration results in a second frequency that is changed by at least about 10% as compared to said first frequency.

3. The polynucleotide for use according to claims 1 or 2, wherein the polynucleotide is a dominant-negative construct.

4. The polynucleotide for use according claim 3, wherein the dominant-negative construct is co-expressed in cells expressing wildtype HCN molecules.

5. The polynucleotide for use according to any one of claims 1 to 4, wherein expression of the polynucleotide suppresses current through HCN1 and/or HCN2-encoded ion channels of the myocardial cells.

6. The polynucleotide for use according to any one of claims 1 to 5, wherein expression of the polynucleotide does not alter gating or permeation properties of unsuppressed HCN1 and/or HCN2 channels of the myocardial cells.

7. The polynucleotide for use according to any one of claims 1 to 6, wherein expression of the polynucleotide after administration is driven by an inducible promoter.

8. The polynucleotide for use according to claim 7, wherein the inducible promoter is regulated by an externally controllable stimulus.

9. The polynucleotide for use according to claim 7, wherein the inducible promoter is regulated by a hormone or cytokine.

10. The polynucleotide for use according to any one of claims 1 to 9, wherein said quiescent myocardial cells have no firing rate prior to said administration and after said administration said myocardial cells generate spontaneous repetitive electrical signals.

11. The polynucleotide for use according to any one of claims 1 to 9, wherein said first frequency of contraction or electrical activity is an inappropriate frequency and said second frequency of contraction or electrical activity is increased or decreased as compared to said first frequency.

12. The polynucleotide for use according to any one of claims 1 to 11, wherein the administration is by injection or perfusion into the myocardium of a mammalian subject.

13. The polynucleotide for use according to any one of claims 1 to 12, wherein the administration is for modulating the contraction function or electrical activity of the myocardial cells of a mammalian subject afflicted by cardiac related syncope, abnormal sinus node function, atrio-ventricular block, or bradycardia-tachycardia syndrome.

14. A polynucleotide encoding an amino acid sequence for a mutant HCN ion channel, wherein said amino acid sequence comprises three alanine molecules at positions 365 to 367 as compared to the sequence of a wild-type HCN1 channel.

15. A vector comprising a polynucleotide according to any claim 14, wherein said vector is suitable for administration to cardiac tissue.

epicardium

100 μm

endocardium

# FIG. 1

FIG. 2D

FIG. 2E

FIG. 2A

FIG. 2B

FIG. 2C

FIG. 2F

FIG. 3B

FIG. 3A

FIG. 3C

FIG. 3D

EP 2 314 306 A1

EP 2 314 306 A1

FIG. 3E

FIG. 3F

FIG. 3G

FIG. 4A

FIG. 4B

EP 2 314 306 A1

EP 2 314 306 A1

Baseline

Cadmium

**FIG. 4C**

**FIG. 4D**

FIG. 4F

FIG. 4E

33

FIG. 5A

FIG. 5B

FIG. 5C

Baseline

72 hours later

100 ms

100 ms

FIG. 6A

72 hours later

100 ms

FIG. 6B

EP 2 314 306 A1

Pore

```
HCN1     380-  SYALFKAMSHMLCIGYGAQAPVS  -402
HCN2     388-  SFALFKAMSHMLCIGYGRQAPES  -410
HCN3     298-  SHALFKAMSHMLCIGYGQQAPVG  -320
HCN4     466-  SYALFKAMSHMLCIGYGRQAPVG  -488
SPIH     416-  TWALFKALSHMLCIGYGKFPPQS  -438
Shaker   430-  PDAFWWAVVTMTTVGYGDMTPVG  -452
KcsA     63 -  PRALWWSVETATTVGYGDLYPVT  - 85
```

GYG

N

C

S4 Voltage Sensor
253

```
HCN1    EVY-KTARALRIVRFTKILSLLRLLRLSRLTRYTH
HCN2    EVY-KATRALRIVRFTKILSLLRLLRLSRLIRYIH
HCN3    EVY-KTARALRIVRFTKILSLLRLLRLSRLIRYMH
HCN4    EVY-KTARALRIVRFTKILSLLRLLRLSRLIRYIH
SPIH    EVS----RALKILRFAKLLSLLRLLRLSRLMRFVS
KAT1    ELGFRILSMLRLWRLRRVSSLFARLEKDIRFN
Shaker  AIL-RVIRLVRVFRIFKLSRHSK
HERG    GLL-KTARLLRLVRVARKLDRYS
```

FIG. 7

EP 2 314 306 A1

WT-HCN1

HCN1-AAA

Uninjected

0mV

-30mV

-150mV

-140mV

10 μA

1 sec

FIG. 8A

FIG. 8B

EP 2 314 306 A1

50nl WT-HCN2

50nl WT-HCN2
+50nl dH₂O

50nl WT-HCN2
+50nl HCN1-AAA

10 µA

1 sec

FIG. 9A

EP 2 314 306 A1

FIG. 9B

FIG. 9C

10 μA

200 ms

FIG. 9D

EP 2 314 306 A1

FIG. 10

FIG. 11A

-30mV

40mV

-140mV

-100mV

FIG. 11B

FIG. 11C

EP 2 314 306 A1

FIG. 11D

50nl WT-HCN2          50nl WT-HCN2          50nl WT-HCN2
                      +50nl dH$_2$O          +50nl HCN1-AAA

10 µA

1sec

## FIG. 12A

Normalized I$_{-140mV}$ & 3sec

1.0

0.8

0.6

0.4

0.2

0.0

*

WT-HCN2          WT-HCN2          WT-HCN2
                 + dH$_2$O          +HCN1-AAA

## FIG. 12B

FIG. 12C

EP 2 314 306 A1

FIG. 12D

FIG. 12E

FIG. 12F

EP 2 314 306 A1

E235A

E235R

6 µA

1s

6 µA

1s

FIG. 13A

FIG. 13B

FIG. 13C

FIG. 13D

Europäisches
Patentamt

European
Patent Office

Office européen
des brevets

# EUROPEAN SEARCH REPORT

Application Number

EP 10 18 0543

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SCHREUER KEVIN D ET AL: "Selective knockout of heterologously expressed human inward rectifier potassium current by a dominant negative mutant", 21 October 1997 (1997-10-21), CIRCULATION, LIPPINCOTT WILLIAMS & WILKINS, US, PAGE(S) I422, XP009142485, ISSN: 0009-7322 * abstract * | 1-15 | INV. A61K38/17 C07K14/705 |
| Y | LALLI M JANE ET AL: "Suppression of KATP currents by gene transfer of a dominant negative Kir6.2 construct", PFLUEGERS ARCHIV EUROPEAN JOURNAL OF PHYSIOLOGY, vol. 436, no. 6, November 1998 (1998-11), pages 957-961, XP002614159, ISSN: 0031-6768 * see results and discussion. * | 1-15 | |
| Y | TINKER ANDREW ET AL: "Regions responsible for the assembly of inwardly rectifying potassium channels", CELL, vol. 87, no. 5, 1996, pages 857-868, XP002614160, ISSN: 0092-8674 * the whole document * * page 861, right-hand column, paragraph 2 * | 1-15 | TECHNICAL FIELDS SEARCHED (IPC) A61K C07K |

-/--

The present search report has been drawn up for all claims

| Place of search | Date of completion of the search | Examiner |
|---|---|---|
| Munich | 10 January 2011 | Rojo Romeo, Elena |

EPO FORM 1503 03.82 (P04C01)

Europäisches
Patentamt

European
Patent Office

Office européen
des brevets

**EUROPEAN SEARCH REPORT**

Application Number

EP 10 18 0543

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PEREZ-GARCIA M TERESA ET AL: "Viral gene transfer of dominant-negative Kv4 construct suppresses an O2-sensitive K+ current in chemoreceptor cells", JOURNAL OF NEUROSCIENCE, vol. 20, no. 15, 1 August 2000 (2000-08-01), pages 5689-5695, XP002614161, ISSN: 0270-6474 * in particular results and discussion * ----- | 1-15 | |
| Y,P | WO 02/19966 A2 (UNIV JOHNS HOPKINS) 14 March 2002 (2002-03-14) * page 22 * * page 25 * ----- | 1-15 | |
| A | SANTORO B ET AL: "IDENTIFICATION OF A GENE ENCODING A HYPERPOLARIZATION-ACTIVATED PACEMAKER CHANNEL OF BRAIN", CELL, CELL PRESS, CAMBRIDGE, NA, US, vol. 93, 29 May 1998 (1998-05-29), pages 717-729, XP002922480, ISSN: 0092-8674, DOI: 10.1016/S0092-8674(00)81434-8 * the whole document * * in particular, Fig. 6 * ----- | 1-15 | TECHNICAL FIELDS SEARCHED (IPC) |

-/--

The present search report has been drawn up for all claims

| Place of search | Date of completion of the search | Examiner |
|---|---|---|
| Munich | 10 January 2011 | Rojo Romeo, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 18 0543

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SANTORO BINA ET AL: "The HCN gene family: Molecular basis of the hyperpolarization-activa ted pacemaker channels", ANNALS OF THE NEW YORK ACADEMY OF SCIENCES, NEW YORK, vol. 868, 30 April 1999 (1999-04-30), pages 741-764, XP002177233, ISSN: 0077-8923, DOI: 10.1111/J.1749-6632.1999.TB11353.X * the whole document * * in particular, Fig. 2. * | 1-15 | |
| T | XUE TIAN ET AL: "Dominant-negative suppression of HCN1- and HCN2-encoded pacemaker currents by an engineered HCN1 construct: Insights into structure-function relationships and multimerization.", CIRCULATION, vol. 106, no. 19 Supplement, 5 November 2002 (2002-11-05), pages II-68, XP002614162, & ABSTRACTS FROM SCIENTIFIC SESSIONS; CHICAGO, IL, USA; NOVEMBER 17-20, 2002 ISSN: 0009-7322 * the whole document * | 1-15 | |
| T | DATABASE PROTEIN [Online] 5 November 2010 (2010-11-05), Santoro et al.: "Increased seizure severity and seizure-related death in mice lacking HCN1 channels.", XP002614163, retrieved from NCBI Database accession no. NP_034538 * the whole document * | 1-15 | TECHNICAL FIELDS SEARCHED (IPC) |

The present search report has been drawn up for all claims

| Place of search | Date of completion of the search | Examiner |
|---|---|---|
| Munich | 10 January 2011 | Rojo Romeo, Elena |

EPO FORM 1503 03.82 (P04C01)

## ANNEX TO THE EUROPEAN SEARCH REPORT
## ON EUROPEAN PATENT APPLICATION NO.

EP 10 18 0543

This annex lists the patent family members relating to the patent documents cited in the above-mentioned European search report.
The members are as contained in the European Patent Office EDP file on
The European Patent Office is in no way liable for these particulars which are merely given for the purpose of information.

10-01-2011

| Patent document cited in search report | | Publication date | Patent family member(s) | | Publication date |
|---|---|---|---|---|---|
| WO 0219966 | A2 | 14-03-2002 | AU | 8880701 A | 22-03-2002 |
| | | | CA | 2421584 A1 | 14-03-2002 |
| | | | EP | 1324780 A2 | 09-07-2003 |
| | | | JP | 2004533405 T | 04-11-2004 |

For more details about this annex : see Official Journal of the European Patent Office, No. 12/82

## REFERENCES CITED IN THE DESCRIPTION

### Patent documents cited in the description

- US 60287088 B
- US 6376471 B
- US 9823877 W, Marban, E.
- US 6214620 B, D.C. Johns and E. Marban
- WO 9522618 A
- US 20020022259 A1
- US 5436128 A
- US 20010024924 A1

### Non-patent literature cited in the description

- The Heart and Cardiovascular System. Scientific Foundations. Raven Press, 1986
- **Bosch, R. et al.** *Cardiovas Res.,* 1999, vol. 44, 121
- **Donahue, J. et al.** *Gene Therapy,* 1998, vol. 5, 630
- **Donahue, J. et al.** *PNAS (USA),* vol. 94, 4664
- **Akhttr, S. et al.** *PNAS (USA),* 1997, vol. 94, 12100
- **Benson, D.A. et al.** *Nucl. Acids. Res.,* 1997, vol. 25, 1
- **Sambrook et al.** Molecular Cloning: A Laboratory Manual. 1989
- **Ausubel et al.** Current Protocols in Molecular Biology. John Wiley & Sons, 1989
- **Wong et al.** *Nature,* 1991, vol. 351 (6321), 63
- **De Jongh KS et al.** *J Biol Chem,* 05 September 1990, vol. 265 (25), 14738
- **Perez-Reyes, E. et al.** *J Biol Chem,* 25 January 1992, vol. 267 (3), 1792
- *Neuroscientist,* February 2001, vol. 7 (1), 42
- **Isom, LL. et al.** *Science,* 08 May 1992, vol. 256 (5058), 839
- **Isom, LL. et al.** *Cell,* 03 November 1995, vol. 83 (3), 433
- **E. Marban.** *J. Gen Physiol.,* August 2001, vol. 118 (2), 171-82
- *Circ Res.,* 20 July 2001, vol. 89 (2), 160-7
- *Circ Res.,* 20 July 2001, vol. 89 (2), 101
- *Circ Res.,* 06 July 2001, vol. 89 (1), 33-8
- *Circ Res.,* 22 June 2001, vol. 88 (12), 1267-75
- *J Biol Chem.,* 10 August 2001, vol. 276 (32), 30423-8
- *Circulation,* 22 May 2001, vol. 103 (20), 2447-52
- *Circulation.,* 15 May 2001, vol. 103 (19), 23634
- *Am J Physiol Heart Circ Physiol.,* June 2001, vol. 280 (6), H2623-30
- *Biochemistry,* 22 May 2001, vol. 40 (20), 6002-8
- *J Physiol.,* 15 May 2001, vol. 533, 127-33
- *Proc Nat1 Acad Sci U S A.,* 24 April 2001, vol. 98 (9), 5335-40
- *Circ Res.,* 30 March 2001, vol. 88 (6), 570-7
- *Am J Physiol Heart Circ Physiol.,* April 2001, vol. 280 (4), H1882-8
- *J Mol Cell Cardiol.,* November 2000, vol. 32 (1 1), 1923-30
- **Gingrich ; Roder.** *Ann. Rev. Neurosci.,* 1998, vol. 21, 377-405
- **Geller, A.I. et al.** *J. Neurochem,* 1995, vol. 64, 487
- **Lim, F. et al.** DNA Cloning: Mammalian Systems. Oxford Univ. Press, 1995
- **Geller, A.I. et al.** *Proc Natl. Acad. Sci.: U.S.A.,* 1993, vol. 90, 7603
- **Geller, A.I. et al.** *Proc Natl. Acad. Sci USA,* 1990, vol. 87, 1149
- **LeGal LaSalle et al.** *Science,* 1993, vol. 259, 988
- **Davidson et al.** *Not. Genet,* 1993, vol. 3, 219
- **Yang et al.** *J. Virol.,* 1995, vol. 69, 2004
- **Kaplitt, M.G. et al.** *Nat. Genet.,* 1994, vol. 8, 148
- **Davis et al.** *Hum Gene Ther,* 1993, vol. 4, 151
- **Sambrook et al.** Molecular Cloning: A Laboratory Manual. Cold Spring Harbor Laboratory press, 1989
- **Mannino ; Gould-Fogerite.** *BioTechniques,* 1988, vol. 6, 682
- **Felgner ; Holm.** *Bethesda Res. Lab. Focus,* 1989, vol. 11 (2), 21
- **Maurer, R.A.** *Bethesda Res. Lab. Focus,* 1989, vol. 11 (2), 25
- **Quantin et al.** *Proc. Natl. Acad. Sci. USA,* 1992, vol. 89, 2581-2584
- **Stratford-Perricadet et al.** *J. Clin. Invest.,* 1992, vol. 90, 626-630
- **Rosenfeld et al.** *Cell,* 1992, vol. 68, 143-155
- Electrophysiologic Testing. Blackwell Science, Inc, 1999

- **I. Herskowitz.** *Nature,* 1987, vol. 329, 219-22
- **D. C. Johns ; H. B. Nuss ; E. Marban.** *J. Biol. Chem.,* 1997, vol. 272, 31598-603
- **U. C. Hoppe ; E. Marban ; D. C. Johns.** *J. Clin. Invest.,* 2000, vol. 105, 1077-84
- **D. C. Johns ; R. Marx ; R. E. Mains ; B. O'Rourke ; E. Marban.** *J Neurosci.,* 1999, vol. 19, 1691-7
- **J. K. Donahue et al.** *Nat Med,* 2000, vol. 6, 1395-8
- **J. K. Donahue et al.** *Nat. Med,* 2000, vol. 6, 1395-8
- **R. Mitra ; M. Morad.** *Proc Nail Acad Sci U SA,* 1986, vol. 83, 5340-4
- **U. C. Hoppe ; D. C. Johns ; E. Marban ; B. O'Rourke.** *Circ Res,* 1999, vol. 84, 964-72
- **U. C. Hoppe ; E. Marban ; D. C. Johns.** *J Clin Invest,* 2000, vol. 105, 1077-84
- **U. C. Hoppe ; E. Marban ; D. C. Johns.** *Proc Natl Acad Sci U S A,* 2001, vol. 98, 5335-40
- **U. C. Hoppe ; E. Marban ; D. C. Johns.** *Proc Natl Accrd Sci U S A,* 2001, vol. 98, 5335-40
- **E. Hayes ; M. K. Pugsley ; W. P. Penz ; G. Adaikan ; M. J. Walker.** *J Pharmcacol. Toxicol. Methods,* 1994, vol. 32, 201-7
- **B. Santoro et al.** *Cell,* 1998, vol. 93, 717-29
- **R. Li et al.** *J. Physiol,* 2001, vol. 533, 127-33
- **J. Seharaseyon.** *J. Mol. Cell Cardiol.,* 2000, vol. 32, 1923-30
- **MT Perez-Carcia.** *J. Neurosci,* 2000, vol. 20, 5689-95
- **J. Seharseyon et al.** *J. Biol Chem.,* 2000, vol. 275, 17561-5
- **UC Hoppe et al.** *J. Clin Invest,* 2000, vol. 105, 1077-84
- **MJ Lalli et al.** *Pflugers Arch.,* 1998, vol. 436, 957-61
- **DC John et al.** *J. Biol Chem,* 1997, vol. 272, 31598-603
- **Hille B.** Ion channels of Excitable Membranes. Sinauer Associates, Inc, 2001
- **R. MacKinnon.** *Nature,* 1991, vol. 350, 232-5